# EUROPEAN PATENT APPLICATION

(11) **EP 2 561 886 A1**
(43) Date of publication of application: **27.02.2013**
(21) Application number: 11178451.8
(22) Date of filing: 23.08.2011
(51) Int. Cl.: A61K 38/17, A61P 23/00, A61P 25/00

(54) **Peptide adjuvant for improved peripheral analgesia**

(71) Applicant: Forschungsverbund Berlin e.V., 12489 Berlin (DE); Julius-Maximilians-Universität Würzburg, 97070 Würzburg (DE)
(72) Inventor: Blasig, Ingolf, 12621 Berlin (DE); Rittner, Heike, 97078 Würzburg (DE); Zwanziger, Denise, 13189 Berlin (DE)
(74) Representative: Lange, Sven

(57) **Abstract**

The invention relates to peptides comprising an amino acid sequence of or derived from a claudin protein for use as a pharmaceutical agent in pain therapy. The invention relates further to claudin protein as a target in pain therapy, in addition to the use of the peptides for opening the perineurium, dissociating the tight junctions of the perineurium and/or increasing paracellular permeability, preferably of ions, small molecules, pharmaceutical agents and/or high or low molecular weight compounds, through the perineurium. The peptides are therefore intended as an adjuvant for painkiller administration, such as analgesics and/or anesthetics, preferably opioids, such as morphine or DAMGO, or sodium channel blockers, such as alkaloid based toxins, for example tetrodotoxin (TTX) and/or saxitoxin (STX), thereby enhancing painkiller function.

## Description

The invention relates to peptides comprising an amino acid sequence of or derived from a claudin protein for use as a pharmaceutical agent in pain therapy. The invention relates further to claudin protein as a target in pain therapy, in addition to the use of the peptides for opening the perineurium, dissociating the tight junctions of the perineurium and/or increasing paracellular permeability, preferably of ions, small molecules, pharmaceutical agents and/or high or low molecular weight compounds, through the perineurium. The peptides are therefore intended as an adjuvant for painkiller administration, such as analgesics and/or anesthetics, preferably opioids, such as morphine or DAMGO, or sodium channel blockers, such as alkaloid based toxins, for example tetrodotoxin (TTX) and/or saxitoxin (STX), thereby enhancing painkiller function.

### BACKGROUND OF THE INVENTION

The paracellular tightness of endothelial and epithelial is formed by transmembrane tight junction (TJ) proteins (1, 2). These integral structures are the most apical type of cell-cell contacts regulating the paracellular flux of various, preferably hydrophilic, molecules. Transmembrane proteins of the TJ are occludin-like proteins, junctional adhesion molecules, and claudins (3, 4). The claudin family contains 24 members of tetraspan membrane proteins with a cytosolic N- and C-terminus as well as two extracellular loops (ECL). Claudins determine the tissue-, charge-, and size-selectivity of the paracellular seal (5, 6). Modulation of paracellular permeation represents a potent tool to enhance penetration of pharmaceutical agents.

Present strategies for the opening of tissue barriers in the clinic are often related to an unspecific mode of action, require drastic efforts, have a low success rate and a high risk for side effects (7-10). Novel substances to enhance the paracellular permeation in a less toxic manner should be specific, selective, and transient modulators of TJ. Such modulators are peptides derived from ECLs of TJ proteins (11-14) and specific ligands thereof (e.g., *Clostridium perfringens* enterotoxin, CPE) (15-18). Recently, a claudin-1 derived peptide which modulates the TJ-integrity of human colonic adenocarcinoma (T84) cells and increases the permeability in the gastrointestinal tract of rats, has been described (14).

Peripheral nerves are surrounded by the perineurium, which functions as a barrier to block unrestricted flux of substances and mediators for nerve protection (19). The perineurium consists of several layers of perineurial cells on a basal membrane. In the embryonic development the perineurium is formed by the neuroectoderm (20). Several TJ proteins are found in the perineurium including claudins and occludin (21). Of these, claudin-1 is strongly expressed in the perineurium of human as well as rat peripheral nerves (21, 22). Supporting the role of claudin-1 in sealing barriers, claudin-1-/- mice die on day 2 after birth because fluids are uncontrollably lost through the skin (23). In the perineurium claudin-1 is lost after nerve injury which correlates with the opening of the barrier (21).

Regional anaesthesia is used perioperatively during surgery and postoperatively for pain control. Currently, suitable drugs in the clinic are limited to lipophilic unspecifically acting local anesthetics (e.g., lidocaine), due to the limited penetration through the perineurium. Ideally, pharmaceutical agents for regional analgesia should only block nociceptors and spare motor and sensory neurons (24). Hydrophilic pharmaceutical agents would have the advantage of lower side effects due to their lack of brain penetration. Analgesic drugs for nociceptive neurons could be opioids (e.g., [D-Ala², N-MePhe⁴, Gly⁵-ol]-enkephalin (DAMGO)) binding to mu-opioid receptors on peripheral nociceptive neurons (7, 25, 26) or voltage gated sodium channel blockers (e.g., tetrodotoxin (TTX)) (10). However, these hydrophilic agents do not easily penetrate the perineurium. So far, only non-specific chemical permeation enhancers have been employed (10).

### SUMMARY OF THE INVENTION

In light of the prior art, the technical problem underlying the present invention is to provide an agent that enhances opening of the perineurium for delivery of pharmaceutical agents.

This problem is solved by the features of the independent claims. Preferred embodiments of the present invention are provided by the dependent claims.

Therefore, an object of the invention is to provide an isolated peptide comprising an amino acid sequence of or derived from a claudin protein for use as a pharmaceutical agent in pain therapy.

In one embodiment the isolated peptide of the present invention is characterised in that the peptide exhibits an amino acid sequence of or derived from the first extra-cellular loop (1. ECL) of a claudin protein.

The invention relates to an isolated peptide, characterised in that the peptide exhibits an amino acid sequence comprising
- a sequence according to SEQ ID NO. 1 (X₁X₂VX₃QSTGX₄X₅QX₆KVX₇DSLLX₈LX₉X₁₀X₁₁R), whereby X₁ to X₁₁ can be any amino acid,
- a sequence according to SEQ ID NO. 1, whereby X₁=S, N; X₂=S, C; X₃=S, V; X₄=Q, H; X₅=l, M; X₆=S, C; X₇=F, Y; X₈=N, A; X₉=S, N, P; X₁₀=S, Q, T and X₁₁=T, D.
- a sequence according to
   SEQ ID NO. 2 (SSVSQSTGQIQSKVFDSLLNLNSTLQATR)
   SEQ ID NO. 3 (SCVSQSTGQIQCKVFDSLLNLSSTR),
   SEQ ID NO. 4 (SCVVQSTGQMQCKVYDSLLALPQDR),
   SEQ ID NO. 5 (SCVVQSTGQMQCKVYDSLLALPQDR),
   SEQ ID NO. 6 (NCVVQSTGQMQCKVYDSLLALPQDR),
   SEQ ID NO. 7 (NCVVQSTGQMQCKVYDSLLALPQDR),
   SEQ ID NO. 8 (SCASQSTGQVQCKLYDSLLALDGHR),
   SEQ ID NO. 9 (SCVVQSTGHMQCKVYDSVLALSTER),
   SEQ ID NO. 10 (DCVTQSTGMMSCKMYDSVLALSAAR),
   SEQ ID NO. 11 (NCVRQANIRMQCKIYDSLLALSPDR) and/or
   SEQ ID NO. 12 (ECVWHSTGIYQCQIYRSLLALPQDR),
- a sequence that exhibits any number of conservative amino acid substitutions in comparison to a sequence according to any one of SEQ ID NO. 1 to 12, and/or
- a sequence that exhibits more than 80%, preferably more than 90%, sequence similarity to a sequence according to any one of SEQ ID NO. 1 to 12.

Peptides of the above-mentioned sequences are in a preferred embodiment intended for use as a pharmaceutical agent in pain therapy.

In a preferred embodiment the isolated peptide of the present invention is characterized in that the peptide comprises a beta-sheet secondary structure.

The invention relates to an isolated peptide as described herein for use in opening the perineurium, dissociating the tight junctions of the perineurium and/or increasing paracellular permeability, preferably of ions, small molecules, pharmaceutical agents and/or high or low molecular weight compounds, through the perineurium. The opening of the perineurium is in a preferred embodiment a transient effect of the inventive peptides. After 24 h post treatment the integrity of the tight junctions (in Caco-2 monolayers) is restored.

The invention relates to an isolated peptide as described herein for use as an adjuvant for enhanced delivery and/or transport of pharmaceutical agents across one or more neurological barriers, preferably perineural delivery of pharmaceutical agents to peripheral nerve cells.

In a preferred embodiment the pharmaceutical agent for perineural delivery is a painkiller, such as one or more analgesics and/or anesthetics, preferably opioids, such as morphine or DAMGO, or sodium channel blockers, such as alkaloid based toxins, for example tetrodotoxin (TTX) and/or saxitoxin (STX).

The invention also relates to an isolated peptide as described herein for use as an adjuvant in the treatment of pain, preferably nociceptive pain.

The isolated peptide of the present invention is also intended for use as an adjuvant for painkiller administration, such as analgesics and/or anesthetics, preferably opioids, such as morphine or DAMGO, or sodium channel blockers, such as alkaloid based toxins, for example tetrodotoxin (TTX) and/or saxitoxin (STX), thereby enhancing painkiller function.

The invention also relates to an isolated peptide as described herein for use as an adjuvant in the treatment of perioperative acute or chronic pain, such as pain associated with joint replacement or other operations involving hips, shoulders, hands, feet and/or knees, complex regional pain syndrome (CRPS) and/or pain associated with ischemic disease, such as peripheral artery occlusive disease

Preferred pain conditions to be treated relate to conditions, where an early mobilisation of a joint or body part after operation is required. For example, after knee or shoulder operations recovery is enhanced by maintaining motion in the joint. Via application of the peptides as described herein (together with a painkiller such as an analgesic) a local pain reduction or elimination can be achieved without hindering the motor skills of the patient. The present invention therefore is particularly relevant for rehabilitation of patients with painful injuries or post-operative wounds who need to maintain mobility. Pain stemming from local sources is therefore also to be treated using the peptides of the present invention.

A further aspect of the invention relates to the isolated peptide of the present invention for use as an adjuvant for enhanced perineural delivery of therapeutic gene vectors across the perineurium, for example enhanced delivery of viral gene vectors, which preferably encode for growth factors or stimulators of cellular regeneration in the treatment of nerve damage. The delivery of said gene vectors, or growth factors themselves, may also be applied both directly in the area of nerve damage in addition to regions surrounding the damage. This particular preferred use of the peptides represents an alternative use to pain therapy. Where neuron-specific, especially peripheral nerve specific expression of therapeutic protein is beneficial in treatment of any given condition, transfer (or transformation) or gene vectors through the perineurium would be of significant help. The peptides of the present invention facilitate opening of the perineurium, thereby facilitation transformation of therapeutic gene vectors into the target cells of interest.

In a further embodiment the present invention relates to claudin protein as a target for pain therapy.

A further aspect of the invention relates to a pharmaceutical composition comprising the peptide as described herein with a pharmaceutically acceptable carrier material.

The pharmaceutical composition of the present invention can also comprise additionally one or more painkillers, such as analgesics and/or anesthetics, preferably opioids, such as morphine or DAMGO, or sodium channel blockers, such as alkaloid based toxins, for example tetrodotoxin (TTX) and/or saxitoxin (STX).

The invention further relates to a kit comprising the peptide of the present invention and/or the pharmaceutical composition as described above in addition to a painkiller, which are physically separated and can be mixed shortly prior to administration.

The invention further relates to an isolated peptide as described herein and/or the pharmaceutical composition as described herein for local and/or regional application, preferably for local treatment of pain in a subject by injection or topical application.

The isolated peptide of the present invention is also intended for use as an adjuvant in the treatment of pain, characterised in that patient motor skills are maintained during pain treatment.

The invention further relates to an isolated peptide as described herein for use in treatment of any medical condition in which opening the perineurium, dissociating the tight junctions of the perineurium and/or increasing paracellular permeability, preferably of ions, small molecules, pharmaceutical agents and/or high or low molecular weight compounds, through the perineurium during treatment, would be of benefit to a patient.

A further aspect of the invention relates to a nucleic acid molecule encoding the amino acid sequence for the peptide as described herein. A further related aspect of the invention is a gene expression vector comprising the nucleic acid molecule encoding the inventive peptide. A further related aspect is therefore also a cell comprising the vector and/or nucleic acid as described above.

The invention also relates to a method for enhanced perineural delivery of pharmaceutical agents to peripheral nerve cells comprising administration of the peptide of the present invention and/or the pharmaceutical composition as described above with a pharmaceutical agent.

The invention also encompasses a method for the treatment of pain comprising administration of an effective amount of the peptide of the present invention and/or the pharmaceutical composition as described above as an adjuvant for painkiller administration, such as administration of analgesics and/or anesthetics, preferably opioids, such as morphine or DAMGO, or sodium channel blockers, such as alkaloid based toxins, for example tetrodotoxin (TTX) and/or saxitoxin (STX), thereby enhancing painkiller function, to a subject in need of pain treatment.

In a preferred embodiment the peptide of the present invention is applied in advance of pain occurrence, for example in the case of operative procedures the peptides could be administered with a pain killer in advance of the operation. The peptides in some delivery or application forms will take some time to provide the intended effect, so pre-emptive treatment is preferred.

### DETAILED DESCRIPTION OF THE INVENTION

The barrier integrity of tight junction (TJ) proteins limits the paracellular flux of substances. Within the family of tight junction proteins, claudin-1 seems to be a key protein for tightness formation and integrity. In the peripheral nervous system the nerve fibers are surrounded by a barrier formed of the perineurium, which expresses claudin-1. To enhance the access of pharmaceutical agents via the paracellular route a claudin-1 derived peptide, C1C2, was designed and tested. C1C2 transiently influences the barrier integrity of Caco-2 cell monolayers in a time- and concentration dependent manner and transiently enhances the antinociceptive efficacy of opioids and TTX *in vivo.* It transiently increased the paracellular permeability for ions, high, and low molecular weight compounds. Structural studies revealed a relevance of β-sheet elements for the functionality of the peptide. C1 C2 structurally shows a higher percentage of β-sheet than the non-functional C2C2.

In vivo perineurial injection of C1C2 in rats facilitated the effect of antinociceptive agents and raised mechanical nociceptive thresholds. The mechanism is related to the internalization of C1C2 and to a vesicle-like distribution within the cells. C1C2 decreased membrane-localized claudin-1 of cells in culture and in vivo in the perineurium of rats after perineurial injection. The peptide internalizes in claudin-1-YFP expressing HEK-293 cells, co-localizes with the claudin-1 protein and reduces the amount of membrane-localized claudin-1. In vivo, expression of claudin-1 in the perineurium is decreased. Therefore claudin-derived peptides, such as C1C2, represent novel agents to transiently open the perineurium for improved delivery of pharmaceutical agents.

The isolated peptides of the present invention relate to peptides with amino acid sequences of or derived from claudin proteins. The term "derived" relates to peptides that exhibit significant structural and/or sequence characteristics in common with claudin protein amino acid sequences, which exhibit the inventive properties of the peptides of the present invention. The "claudin proteins" relates to proteins from the claudin family, which contains various members of tetraspan membrane proteins with a cytosolic N- and C-terminus as well as two extracellular loops (ECL). The claudin family members can be determined using sequence similarity-based approaches and are known in the art (1-6).

Sequence similarity relates to a measure of the similarity of amino acid or nucleotide sequences. The similarity is commonly measured via sequence alignment and subsequent analysis of shared amino acids at any or all positions across the sequence. For example, 85% similarity means that 85 amino acid positions out of 100 are identical in the two amino acid sequences. Various computational approaches are available to those in the art to determine sequence similarity, such as BLAST, or other sequence alignment methods.

Sequence variants of the claimed peptides that maintain the said properties of the invention are also included in the scope of the invention. Modifications which occur through substitutions are included within the scope of the invention. Substitutions as defined herein are modifications made to the amino acid sequence of the protein or peptide, producing a protein or peptide which contains a different amino acid sequence than the primary protein or peptide without significantly altering the function of the protein. Like additions, substitutions may be natural or artificial. It is well known in the art that amino acid substitutions may be made without significantly altering the peptide's function. This is particularly true when the modification relates to a "conservative" amino acid substitution, which is the substitution of one amino acid for another of similar properties. Such "conserved" amino acids can be natural or synthetic amino acids which because of size, charge, polarity and conformation can be substituted without significantly affecting the structure and function of the peptide. Frequently, many amino acids may be substituted by conservative amino acids without deleteriously affecting the protein's function.

In general, the non-polar amino acids Gly, Ala, Val, Ile and Leu; the non-polar aromatic amino acids Phe, Trp and Tyr; the neutral polar amino acids Ser, Thr, Cys, Gln, Asn and Met; the positively charged amino acids Lys, Arg and His; the negatively charged amino acids Asp and Glu, represent groups of conservative amino acids. This list is not exhaustive. For example, it is well known that Ala, Gly, Ser and sometimes Cys can substitute for each other even though they belong to different groups. Conservative amino acid substitutions are not limited to naturally occurring amino acids, but also include synthetic amino acids. Commonly used synthetic amino acids are omega amino acids of various chain lengths and cyclohexyl alanine which are neutral non-polar analogs; citrulline and methionine sulfoxide which are neutral non-polar analogs, phenylglycine which is an aromatic neutral analog; cysteic acid which is a positively charged analog and ornithine which is a positively charged amino acid analog. Like the naturally occurring amino acids, this list is not exhaustive, but merely exemplary of the substitutions that are well known in the art.

Further, alternatively or additionally, one, two or even three amino acid residues may be replaced by other amino acid residues. These amino acid residues may be natural amino acid residues or non-natural amino acids. Moreover, alternatively or additionally, one, two or even three amino acid residues may be inserted into the sequence of said peptides, wherein these amino acid residues may be natural amino acid residues or non-natural amino acids.

Optionally, the peptides of the present invention may be lipidated, phosphorylated, sulfated, cyclized, oxidated, reduced, decarboxylated, acetylated, acylated, amidated, deamidated, pegylated, biotinylated or bound to one or more other small molecule(s) and/or terpene(s). The peptide may also be conjugated or non-covalently associated to molecular structures that may facilitate uptake such as, e.g., positively charged polymers (e.g., polyethylene imine (PEI)), neutral lipids, positively charged lipids, viral vectors, liposomes, micro- and/or nanobeads, cell-penetrating peptides (= protein-transduction domains, e.g., polyarginines (e.g., R8, R9, R10), HIV Tat peptide, antennapedia/penetratin peptide, a Lactoferrin-derived peptide), antimicrobial peptides and/or a Chariot peptide. Preferably, the peptide bears a free amino terminus and a free carboxy terminus or may have a blocked amino terminus and/or carboxy terminus. Exemplarily, the amino terminus may be acylated or acetylated and/or the carboxy terminus may be amidated.

Preferably, the peptide consists of natural L-amino acids. However, the peptide may also comprise one or more non-natural amino acid(s) such as, e.g., D-amino acid(s), beta amino acid(s), methylated amino acid(s) (e.g., N-methylated amino acid(s)). The peptide may even only consist of non-natural amino acids. The peptide may also be a *retro-inverso* peptide, thus, a peptide mainly comprising D-amino acids and a reverse amino acid sequence compared to the corresponding peptide mainly comprising L-amino acids.

The peptide may be used as an unbound molecule or may be non-covalently formulated with and/or conjugated to any other molecules such as, e.g., high-molecular weight compounds. Optionally, the peptide of the present invention may be stabilized against degradation by conjugation to one or more high-molecular weight compound(s).

Exemplarily, the peptide may be conjugated to one or more hydrophilic polymer(s). The term "hydrophilic polymer" as used herein may be understood in the broadest sense as any polymeric molecule that has a hydrophilic surface. A polymeric molecule comprises more than one monomer. The hydrophilic polymer may have a molecular mass of more than 500 Da. The hydrophilic polymer may be a soluble polymer, a gel-like polymeric matrix, a bead, a surface, a bead coating or the coating of a surface. Preferably, the hydrophilic polymer of the present invention is a soluble polymer. Exemplarily, the hydrophilic polymer of the present invention may be polyethylene glycol (PEG) or derivatives thereof, polyethylene imine (PEI) or derivatives thereof, polyacrylic acid or derivatives thereof, in particular hydroxypropyl methacrylate (HPMA), polysaccharide(s) or derivatives thereof, preferably amino polysaccharide(s), aminoalkyl polysaccharide(s), hydroxyalkyl polysaccharide(s) and/or alkyl polysaccharide(s), lipopolysaccharide(s), hydrophilic polypeptide(s) and/or conjugate(s) or blockpolymer(s) comprising two or more of the aforementioned. Alternatively, the hydrophilic polymer may be a bead having a hydrophilic surface or a bead having a coating comprising a hydrophilic polymer, in particular wherein the bead is a micro- or a nanobead. The hydrophilic polymer of the present invention may be conjugated with the peptide of the present invention via any functional group of the peptide. In particular the hydrophilic polymer may be conjugated with the N-terminus of the peptide, an amino acid residue side chain, or the C-terminus of the peptide.

A "nucleic acid molecule" as used herein may be any polynucleotide known in the art. The nucleic acid molecule may be linear or circular. Preferably, the nucleic acid molecule is double-stranded deoxyribonucleic acid (DNA). The double-stranded DNA may be linear or circular. The double-stranded DNA may be a plasmid. Alternatively, the nucleic acid molecule may also be single-stranded DNA, ribonucleic acid (RNA) or a nucleic acid molecule composed of nucleic acid analogues, such as, e.g., a peptide nucleic acid (PNA) nucleotide, a Morpholino nucleotide, glycol nucleic acid (GNA) nucleotide, a threose nucleic acid (TNA) or a methylated DNA nucleic acid molecule. It will be understood by a person skilled in the art that different types of nucleic acid molecule may also be combined with another. An "expression vector" according to the present invention is any nucleic acid molecule encoding the peptide of the present invention, in addition to exhibiting other DNA features necessary for expression, such as a promoter. Expression vectors are known to those skilled in the art. A "therapeutic gene vector" relates to an expression vector that encodes a therapeutic protein. Administration of a therapeutic vector leads to expression of the therapeutic protein from the transformed cells and subsequent therapeutic effect.

The "pharmaceutical agents" of the present invention relate to any compound or molecule that is intended to show a pharmacological, biological, diagnostic, therapeutic and/or preventative effect after administration to a subject. The peptides of the present invention are intended to be used as a pharmaceutical agent, in order to improve paracellular permeability, preferably through the perineurium. The peptides exhibit a pharmacological and/or biological effect upon the tight junctions, resulting in increased permeability of other molecules. The peptides are therefore useful as adjuvants for paracellular delivery of further pharmaceutical agents of any kind. These further pharmaceutical agents are preferably painkillers. The further agent itself is not a limiting factor of the invention.

Preferred pharmaceutical agents of the present invention relate to painkillers. "Painkillers" as described herein relate to any agent that has a preventative or alleviating effect against any kind of pain in a subject. The painkillers may as an example be analgesics and/or anesthetics, preferably opioids, such as morphine or DAMGO, or sodium channel blockers, such as alkaloid based toxins, for example tetrodotoxin (TTX) and/or saxitoxin (STX). The painkillers to be used as pharmaceutical agents are not a limiting aspect of the present invention.

A "small molecule" as described herein is typically a low molecular weight organic compound. Small molecules and "low molecular weight compounds" are terms than are often used interchangeably, and relate often pharmacologically active molecules, that may bind with high affinity to biopolymers such as proteins. Small molecules are generally smaller than approximately 800 Daltons. "High molecular weight compounds" relate generally to any compound larger than 800 Daltons, as example these may be biopolymers, such as proteins, peptides, nucleic acid molecules, or other polymers. "Ions" relate to atoms or molecules with a net positive or negative charge.

The term "pain" relates to pain as commonly understood by one skilled in the medical or pharmaceutical fields. The therapy or treatments of all kinds of pain are encompassed by the present invention. Pain can include nociceptive pain, which is caused by stimulation of peripheral nerve fibres. Nociceptive pain includes, but is not limited to acute pain caused by thermal, mechanical and chemical sources. Nociceptive pain may also be divided into visceral, deep somatic and superficial somatic pain. Pain also includes inflammatory pain, which is caused by inflammation in the peripheral tissue e.g. postoperative pain or arthritis. In contrast, neuropathic pain is caused by damage or disease affecting any part of the nervous system involved in bodily feelings (the somatosensory system). Peripheral neuropathic pain can be but is not limited to burning, tingling, electrical and/or stabbing pain.

Preferred conditions to be treated using the present invention relate to acute and chronic pain, such as pain associated with joint replacement or other operations involving hips, shoulders, hands, feet and/or knees, complex regional pain syndrome (CRPS) and/or pain associated with ischemic disease, such as peripheral artery occlusive disease. These conditions do not represent a limiting definition of the conditions to be treated.

In the present invention "treatment" or "therapy" generally means to obtain a desired pharmacological effect and/or physiological effect. The effect may be prophylactic in view of completely or partially preventing a disease and/or a symptom, or may be therapeutic in view of partially or completely curing a disease and/or adverse effect of the disease. In the present specification, "therapy" includes arbitrary treatments of diseases or conditions in mammals, in particular, humans, for example, the following treatments (a) to (c): (a) Prevention of onset of a disease, condition or symptom in a patient; (b) Inhibition of a symptom of a condition, that is, prevention of progression of the symptom; (c) Amelioration of a symptom of a condition, that is, induction of regression of the disease or symptom.

The term "pharmaceutical composition" refers to a combination of the agent as described herein with a pharmaceutically acceptable carrier. The phrase "pharmaceutically-acceptable" refers to molecular entities and compositions that do not produce a severe allergic or similar untoward reaction when administered to a human. As used herein, "carrier" includes any and all solvents, dispersion media, vehicles, coatings, diluents, antibacterial and antifungal agents, isotonic and absorption delaying agents, buffers, carrier solutions, suspensions, colloids, and the like. The use of such media and agents for pharmaceutical active substances is well known in the art. Supplementary active ingredients can also be incorporated into the compositions. A pharmaceutical composition of the present invention can include pharmaceutically acceptable salts of the components therein.

The preferred use of the peptides described herein relates to their application in regional analgesia, preferably together with one or more painkillers. The pharmaceutical composition is therefore intended to encompass compositions and application forms suitable for local or regional application, such as topical application and/or injection.

The pharmaceutical compositions may be in the form of a sterile injectable aqueous or oleagenous suspension. This suspension may be formulated according to the known art using those suitable dispersing or wetting agents and suspending agents known in the art. The sterile injectable preparation may also be in a sterile injectable solution or suspension in a non-toxic parenterally-acceptable diluent or solvent, for example as a solution in 1, 3-butane diol. Among the acceptable vehicles and solvents that may be employed are, amongst others, water, ringer's solution and isotonic sodium chloride solution. In addition, sterile, fixed oils are conventionally employed as a solvent or suspending medium.

In certain circumstances it will be desirable to deliver the pharmaceutical compositions disclosed herein parenterally, intravenously, intramuscularly, subcutaneously or even intraperitoneally. The invention further relates, by way of example, to the following methods of pain therapy, including regional application of the peptides as described herein with painkillers, such as when analgesia is performed, where surgery is or is not contemplated. The invention may also be applied as an adjunct to other anaesthesia, such as local or general anaesthesia. This is suitable for a wide variety of surgery, for example orthopaedic surgery such as hip replacement, general surgery and vascular surgery. The invention may also be applied as a sole approach for surgical anaesthesia, for example where the patient would remain awake during the operation, and/or for post-operative analgesia, after an operation where the invention was applied, used as either the sole anaesthetic, or was used in combination with other anaesthesia. The invention may be applied in the treatment of back pain, whereby local application, such as injection or topical application may improve some forms of back pain.

Solutions of the active compounds as free bases or as pharmacologically acceptable salts may be prepared in water suitably mixed with a surfactant, such as hydroxypropylcellulose. Dispersions may also be prepared in glycerol, liquid polyethylene glycols, and mixtures thereof and in oils. Under ordinary conditions of storage and use, these preparations contain a preservative to prevent the growth of microorganisms.The pharmaceutical forms suitable for injectable use include sterile aqueous solutions or dispersions and sterile powders for the extemporaneous preparation of sterile injectable solutions or dispersions. In many cases, it will be preferable to include isotonic agents, for example, sugars or sodium chloride in the solution. For example, one dosage may be dissolved in 1 ml of isotonic NaCl solution and either added to 1000 ml of hypodermoclysis fluid or injected at the proposed site of infusion. Some necessary variation in the dosage will occur depending on the condition of the subject being treated. The person responsible for administration will, in any event, determine the appropriate dose for the individual subject.

Topical administration is accomplished via a topically applied cream, gel, rinse, etc. containing a peptide. Transdermal administration is accomplished by application of a cream, rinse, gel, etc. capable of allowing the peptide agent, and preferably painkiller, to penetrate the skin and pass the desired biological barriers, such as the perineurium. Compositions of peptide containing compounds suitable for topical application include, but are not limited to, physiologically acceptable implants, ointments, creams, rinses, and gels. Any liquid, gel, or solid pharmaceutically acceptable base in which the peptides are at least minimally soluble is suitable for topical use in the present invention. Compositions of the peptide agents suitable for transdermal administration include, but are not limited to, pharmaceutically acceptable suspensions, oils, creams, and ointments applied directly to the skin or incorporated into a protective carrier such as a transdermal device ("transdermal patch").

Dosage levels of the order of from about 0.01 mg to about 500 mg per kilogram of body weight per day are useful in the treatment of the above-indicated conditions. For example, pain may be effectively treated by the administration of from about 0.01 to 50 mg of the inventive peptide per kilogram of body weight per day (about 0.5 mg to about 3.5 g per patient per day). The amount of active ingredient, either peptide or painkiller, that may be combined with the carrier materials to produce a single dosage form will vary depending upon the host treated and the particular mode of administration. For example, a formulation intended for the oral administration of humans may vary from about 5 to about 95% of the total composition. Dosage unit forms will generally contain between from about 1 mg to about 500 mg of active ingredient. It will be understood, however, that the specific dose level for any particular patient will depend upon a variety of factors including the activity of the specific compound employed, the age, body weight, general health, sex, diet time of administration, route of administration, rate of excretion, drug combination and the severity of the particular disease undergoing therapy. The dosage effective amount of compounds according to the invention will vary depending upon factors including the particular compound, toxicity, and inhibitory activity, the condition treated, and whether the compound is administered alone or with other therapies. Typically a dosage effective amount will range from about 0.0001 mg/kg to 1500 mg/kg, more preferably 1 to 1000 mg/kg, more preferably from about 1 to 150 mg/kg of body weight, and most preferably about 50 to 100 mg/kg of body weight. The invention relates also to a process or a method for the treatment of the abovementioned pathological conditions. The compounds of the present invention can be administered prophylactically or therapeutically, preferably in an amount that is effective against the mentioned disorders, to a warm-blooded animal, for example a human, requiring such treatment, the compounds preferably being used in the form of pharmaceutical compositions.

The invention further relates to peptides of the sequences as described in Table 1, which represent the human sequences of the first extra-cellular loop (1. ECL) of the classical claudins, especially the so-called "tightening" claudins. These particular sequences do not represent a limiting description of the invention. SEQ ID NO. 1 is in one embodiment characterised in that X₁ to X₁₁ can be any amino acid, or that X₁=S, N; X₂=S, C; X₃=S, V; X₄=Q, H; X₅=l, M; X₆=S, C; X₇=F, Y; X₈=N, A; X₉=S, N, P; X₁₀=S, Q, T and X₁₁=T, D. The amino acids for each X substituent are intended as possible variables at each position. For example X₁ can be S or N. Peptides comprising the sequences provided in Table 1 are also encompassed within the invention, so that further sequences, that exhibit for example further X amino acids on either side of the sequences provided in the table, are also included in the scope of the invention.

**Table1. Sequences of the present invention.**

| SEQ ID NO. | Amino acid sequence | Description |
|---|---|---|
| SEQ ID NO. 1: | X₁X₂VX₃QSTGX₄X₅QX₆KVX₇DSLLX₈LX₉X₁₀X₁₁R | Claudin |
| SEQ ID NO. 2: | SSVSQSTGQIQSKVFDSLLNLNSTLQATR | C1C2 |
| SEQ ID NO. 3: | SCVSQSTGQIQCKVFDSLLNLSSTR | Claudin-1, 1. ECL |
| SEQ ID NO. 4: | SCVVQSTGQMQCKVYDSLLALPQDR | Claudin-6, 1. ECL |
| SEQ ID NO. 5: | SCVVQSTGQMQCK\/YDSLLALPQDR | Claudin-9, 1. ECL |
| SEQ ID NO. 6: | NCVVQSTGQMQCKVYDSLLALPQDR | Claudin-3, 1. ECL |
| SEQ ID NO. 7: | NCVVQSTGQMQCKVYDSLLALPQDR | Claudin-4, 1. ECL |
| SEQ ID NO. 8: | SCASQSTGQVQCKLYDSLLALDGHR | Claudin-19, 1. ECL |
| SEQ ID NO. 9: | SCVVQSTGHMQCKVYDSVLALSTER | Claudin-5, 1. ECL |
| SEQ ID NO. 10: | DCVTQSTGMMSCKMYDSVLALSAAR | Claudin-7, 1. ECL |
| SEQ ID NO. 11: | NCVRQANIRMQCKIYDSLLALSPDR | Claudin-8, 1. ECL |
| SEQ ID NO. 12: | ECVWHSTGIYQCQIYRSLLALPQDR | Claudin-14, 1. ECL |
| SEQ ID NO. 13: | ESATHSTGITQSDIYSTLLGLPADIQAAQ | C2C2 |

The invention relates furthermore to claudin proteins, preferably claudin-1, as a target in pain therapy. The use of claudin as a target therefore includes the modulation of claudin function, so that the effect described herein (opening of the perineurium), via various methods or agents, also falls within the scope of the invention. In light of the disclosure provided herein further agents that modulate, preferably inhibit, the claudin proteins, especially claudin-1, in a similar manner to the peptides as described herein, which represent one preferred agent in the modulation of claudin protein, for the purpose of pain alleviation also fall under the scope of the invention.

Examples of such claudin-modulating agents of the present invention include, but are not limited to, claudin peptides as described herein, including polypeptides, peptides, peptidomimetics or functional fragments thereof; nucleic acids which encode a relevant claudin polypeptide or functional fragment thereof, agents which decrease the expression of endogenous claudin, e.g., an agent which decreases transcription of a relevant claudin protein, preferably claudin-1, also small molecules which bind to claudin proteins and modulate their activity, such as antagonist anti-claudin antibody molecules. Antibody inhibitors of claudin are also included in the scope of the present invention. Examples of small molecules include, but are not limited to, peptides, peptidomimetics (e.g., peptoids), amino acids, amino acid analogs, polynucleotides, polynucleotide analogs, nucleotides, nucleotide analogs, organic and inorganic compounds (including heterorganic and organomettallic compounds). Oligonucleotides that either code for and/or reduce claudin function are encompassed in the invention, in addition to anti-sense nucleotides that recognise claudin sequences, resulting in reduction and/or inhibition of claudin, preferably claudin-1, function. RNAi based approaches using antisense nucleic acids that modulate activity and/or expression of the claudin target are included in the scope of the present invention. Such approaches are known to those skilled in the art and can be carried out based on the disclosure of the present invention included herein.

The present invention also discloses methods for assessing the effect of claudin modulation, so that potential molecules that exhibit the effect described herein on perineurium function, can be tested and assayed in order to determine their precise properties. Such approaches can be carried out using high-throughput experimentation, testing libraries for potential molecules that effect claudin protein as a target in the context of pain therapy. Such libraries of molecules relate to any collection of potential modulator molecules described above.

Claudin-1 is responsible for barrier integrity and formation of TJ-strands between epithelial cells. This is supposed to be achieved by *trans*-interactions of ECLs of the claudins between opposing cell membranes (27). Claudin-1 is also expressed in the perineurium surrounding peripheral nerves (22). The transient modulation of these interactions with peptides provides a useful tool for diagnostic and pharmacological efforts. Several groups investigated e.g., occludin-derived peptides (13, 28) or specific ligands of claudins (e.g., CPE) (18, 29) influencing ECL interaction for drug delivery enhancement. The present invention relates however to peptides derived from the ECL1 of claudin-1 as drug enhancer for regional pain therapy. We found claudin-1⁵³⁻⁸¹ with substitutions of Cys⁵⁴ and Cys⁶⁴ by serine and a C-terminal amidation as functional active peptide and the minimal active peptide sequence claudin-1⁵³⁻⁷⁶-R containing the same substitutions, as described by the sequences according to the present invention. These substitutions simplify synthesis and avoid undesirable side reactions. Recently, it has been shown that the cysteines are not required for a similar claudin-1 derived peptide (14), whereas cysteines are necessary for the TJ tightness of the whole claudin (30). In our functional assays we found a *in vitro* and *in vivo* functionality of the modified C1C2. Therefore, the substitutions did not seem to interfere.

The investigation of the structural behaviour of peptides can give important hints for the functionality. The ECL1 of claudins consists of ~50 amino acids and is directly involved in the barrier function (30). Negative and positive charges in ECL1 may contribute to paracellular pore formation in TJ identified by mutation studies (6, 31). Secondary structure predictions resulted in a propensity of the ECL1 to form Beta-strands as well as helical segments (27). Otherwise, the reduced size of the peptides used here could avoid specific interactions between side chains or the formation of a defined secondary structure. Our CD-data pointed to a partly Beta-sheet conformation of C1 C2 in SDS indicating the significance of such a strand for the functionality. Furthermore, the N-terminal labelling of C1C2 is not affecting the structural behaviour. However, a defined secondary structure was only present with additives like SDS or TFE. That means, a flexible structure is given and for the formation of the functional relevant secondary structure a near-closed interaction partner is required in the biological environment.

C1C2 influences established TJ of Caco-2 cells. It decreases the TER and increases the paracellular permeation of high and low molecular weight compounds in a time and concentration dependent manner. A significant influence was observed 24 h after peptide treatment. Most likely, this long time interval is caused by an indirect interruption of claudin-claudin-interactions after internalization of C1 C2. The mechanism is different from the interaction of, e.g., the direct binding of CPE to the ECL2 of some other claudin-subtypes, where the effect begins after 12 to 16 h of CPE administration (18).

Different efficacies were found between the apical and bilateral treatment. This could be explained by the presence of peptidases which are mainly localized on the apical cell membrane (32). It leads to a fast proteolytic degradation followed by non-functionality of C1C2 cleavage products. To optimize the pharmacokinetic profile of C1C2, specific modifications using non-proteinogenic amino acids or D-amino acids can be introduced (33). Another aspect of the invention relates therefore to PEGylation or the introduction of a fatty acid to the peptides described herein (34, 35). Then, an apical treatment without a loss in efficacy could be achieved. These modifications can be introduced N-terminally as we could show that blocking of this terminus is unaffected. Such modifications are commonplace for those skilled in the art and fall within the scope of the present invention. Importantly, TJ-integrity is re-arranged after removal of the peptide which shows reversibility of the effect.

Caco-2 cells express several TJ proteins. Due to the direct involvement of claudin-1 on barrier integrity and formation, the C1C2 efficacy could be specifically related to this claudin-subtype. To clarify the specificity of the peptide, we investigated the cellular pathway of the fluorescently labelled peptide on selective claudin-1-YFP expressing HEK-293 cells. The internalization of C1C2 demonstrates an indirect modulation of the paracellular permeation. We could observe intracellular co-localization of the peptide and claudin-1 in vesicle-like structures. The integration of the peptide into the membrane 24 h post treatment is also in agreement with the late effect.

Immunofluorescence data on Caco-2 cells show a decrease of membrane-localized claudin-1 24 h after peptide treatment. Recently, similar observations were found for the treatment of T84 cells with a claudin-1 derived peptide. Cells were loosing their cell-cell contacts formed by claudin-1 which resulted in an increased paracellular permeation (14). These results are independent of a cell system expressing only one TJ protein or a complete TJ-network. The unspecific internalization of the peptide into HEK-293 wild type cells could be due to the hydrophobicity of the peptide, which allows membrane permeability (36). However, peptide function is the alteration of TJ distribution by decreasing the amount of claudin-1 in the cell membrane.

In the peripheral nerve, the perineurium forms a barrier that prevents the unrestricted flux of mediators or pharmaceutical agents. In addition, the peripheral nerve is protected from mediators or substances in the blood stream by the blood nerve barrier formed by endothelial cells of the vasa vasorum within the nerve. For regional analgesia, the barrier of the perineurium is important. In the past, approaches to open this barrier in ex vivo nerve preparations have been hypertonic solutions (37) or cholic acid (38). Both are using high mM concentrations and are non specific and therefore not suitable in the clinic. The ideal opener for the perineurium is specific and transient, because the barrier also protects the nerve. Using perineurial application of C1C2, pharmaceutical agents like hydrophilic opioids or TTX are able to increase mechanical nociceptive threshold. These agents have no effect when applied perineurially in naive rats (39). Interestingly the effect of C1C2 is transient, which supports the reversibility of the effect.

In the perineurium, claudin-1 and claudin-5 seem to be the relevant TJ proteins for paracellular tightness. In humans in chronic inflammatory polyneuropathy, claudin-5 expression is decreased (40). In rats under pathophysiological conditions like nerve crush injury, the expression of claudin-1 is lost, which correlates with an opening of the perineurial barrier (21). However, a direct link between barrier formation and claudin-1 expression has not yet been shown in the perineurium. The novel peptides of the present invention reduce claudin-1 in the membrane of the perineurium.

At the time of the invention, only lipophilic local anesthetics are used in the clinic in regional anesthesia which block all nerve fibers. Different approaches have been used to reach the goal of nociceptor specific analgesia. For example the non specific hydrophilic local anesthetic QX-314 (active substance) can be applied together with agonists to the TRPV1 channel (enhancer) which is only expressed on nociceptors. The TRPV1 agonist opens an ion channel which allows QX-314 to penetrate only into the nociceptor (24). Alternatively, nociceptor specific substances like opioids or certain sodium channel blockers besides of TTX could be used. New approaches for nociceptor specific analgesia are the combination with permeation enhancer like surfactants for these active substances (48). The present invention relates to peptides that show specific interaction with TJ proteins and that can open the perineurium to facilitate the delivery of pharmaceutical agents that could not be used in the past.

### FIGURES

The invention is further described by the figures, which represent various embodiments of the invention. The figures are not intended to limit the scope of the invention.

### Brief description of the figures:

- Figure 1: Design and structural behaviour of C1C2.
- Figure 2: C1C2 transiently modulates the paracellular permeability of Caco-2 (human epithelial colorectal adenocarcinoma) cell monolayers.
- Figure 3: The perineurium of the sciatic nerve expresses claudin-1 and is modulated by C1C2 to permit opioid antinociception in rats.
- Figure 4: Internalization of TAMRA-C1C2.
- Figure 5: C1C2-induced decrease of membrane-localized claudin-1.
- Figure 6: Perineurial C1C2 treatment decreases claudin-1 in the perineurium of rats.
- Figure 7: Structural Behaviour of C2C2.
- Figure 8: TAMRA-C1C2 but not C2C2 transiently modulate the paracellular permeability of Caco-2 cell monolayers.
- Figure 9: No increase in the permeation of FD-1 0 nor Lucifer Yellow of Caco-2 cell monolayers by C2C2.

### Detailed description of the figures:

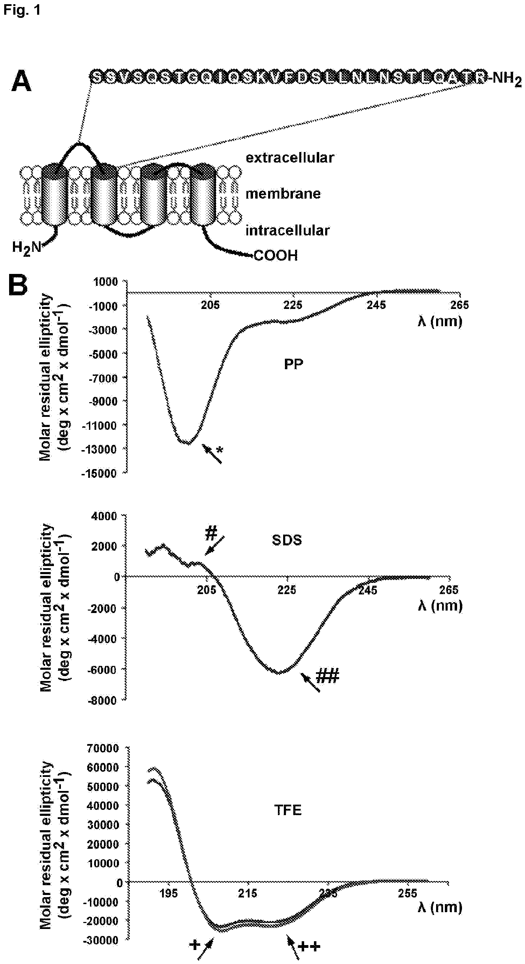

**Fig. 1** **Design and structural behavior of C1C2.** (A) The tight junction protein claudin-1 contains four transmembrane helices, an intracellular located N- and C-terminus as well as two extracellular loops (ECL). C1C2 is derived from the C-terminal part of the ECL1, rat claudin1.53-81. The three mutations, C⁵⁴S⁵⁴, C⁶⁴S⁶⁴, and R(COOH)⁸¹R(CONH₂)⁸¹ are marked in red. (B) CD (circular dichroism) spectroscopy of C1C2 (50 µM) in different solvents. * Random coil of C1C2 in phosphate puffer (PP, 10 mM, pH 7.4) with the characteristic minimum at ~200 nm. #/## ß-sheet folding of C1 C2 in PP and 0.5 mM SDS (sodium dodecyl sulphate) with the characteristic maximum at 190-200 nm and the minimum at 225-210 nm. +/++ helical conformation of C1 C2 in PP and 60% and 80% TFE (2,2,2-trifluoroethanol) with the characteristic minima at 222 and 210-208 nm.

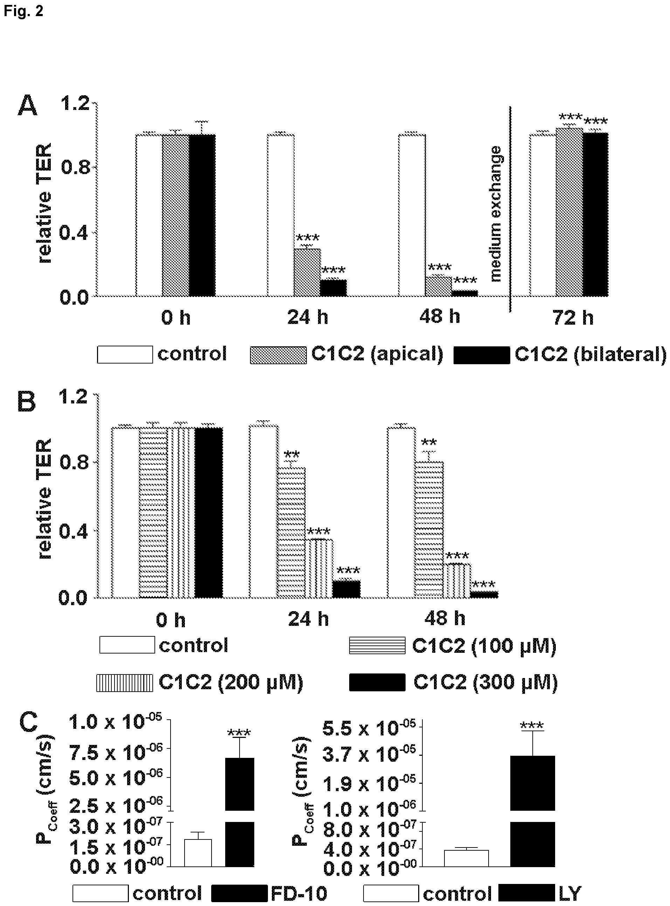

**Fig. 2** **C1C2 transiently modulates the paracellular permeability of Caco-2 (human epithelial colorectal adenocarcinoma) cell monolayers.** (A-C) Cells were grown on 24-well filters (A) Time-dependent reduction of the relative TER after bilateral and apical application of 300 µM C1 C2 followed by the re-arrangement of the cell monolayer after removing of C1 C2 (at 48 h). The bilateral C1C2 treatment results in a higher reduction of the TER as compared to the apical C1C2 treatment. (B) Time- and concentration-dependent reduction of the TER after bilateral application of 100, 200, and 300 µM C1 C2. (C) Determination of the P_{Coeff} (pemeation coefficient; cm/s) of FD-10 (10 kDa) (left) and Lucifer Yellow (LY) (0.4 kDa) (right) of control treated and C1 C2 treated cell monolayers. The paracellular permeability was determined 48 h after C1 C2 administration. Increase of the paracellular permeation of FD-1 0 and Lucifer Yellow by C1C2. Data are represented as mean ± SEM; n=6; ***p< 0.0001 (Mann Withney test, one-tailed) in which in (A, B) 24 h and 48 h are relating to 0 and 72 h based on 48 h.

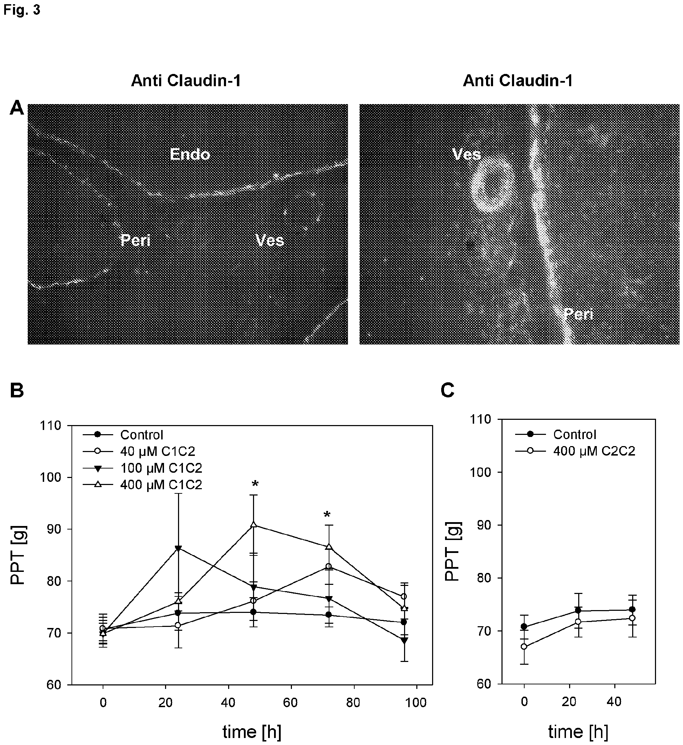

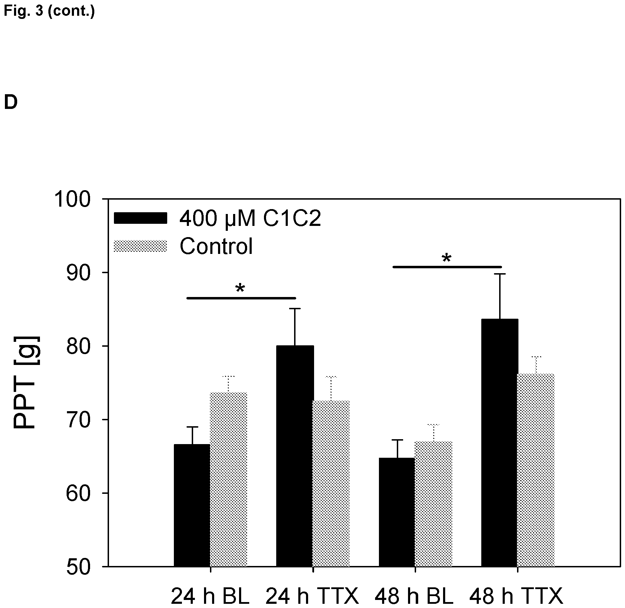

**Fig. 3** **The perineurium of the sciatic nerve expresses claudin-1 and is modulated by C1C2 to permit opioid antinociception in rats.** (A, B) Immunohistochemistry of the sciatic nerve of rats: Immunreactivity of claudin-1 (green) is observed in a cross section of the inner layer of the perineurium (Peri) in two adjacent nerve fascicles as well as in an adjacent blood vessel (Ves). No staining was observed in the endoneurium (Endo) (A 20 X, B 40 X). (C) Rats were injected perineurially with C1 C2 (40-400 µM) or C2C2 (400 µM). Paw pressure thresholds (PPT, g) were obtained before (see results) and 10 min after injection of the opioid receptor agonist [D-Ala², N-MePhe⁴, Gly⁵-ol]-enkephalin (DAMGO, 30 µg). Measurements were obtained before and up to 96 h after injection of C1 C2 or C2C2. An opioid-induced increase in mechanical nociceptive thresholds (PPT) was observed only after C1 C2 but not after C2C2 treatment after 2 days post injection of the claudin peptide (mean ± SEM, n=6, * p<0.05, Two Way ANOVA). (D) Rats were treated as described above for 24 or 48 h with C1 C2 (black bars). Control animals received solvent only (grey bars). Baseline nociceptive thresholds (BL) were obtained, tetrodotoxin (TTX, 10 µM) was injected perineurially and PPT taken afterwards (mean ± SEM, n=6, * p<0.05, Two Way ANOVA).

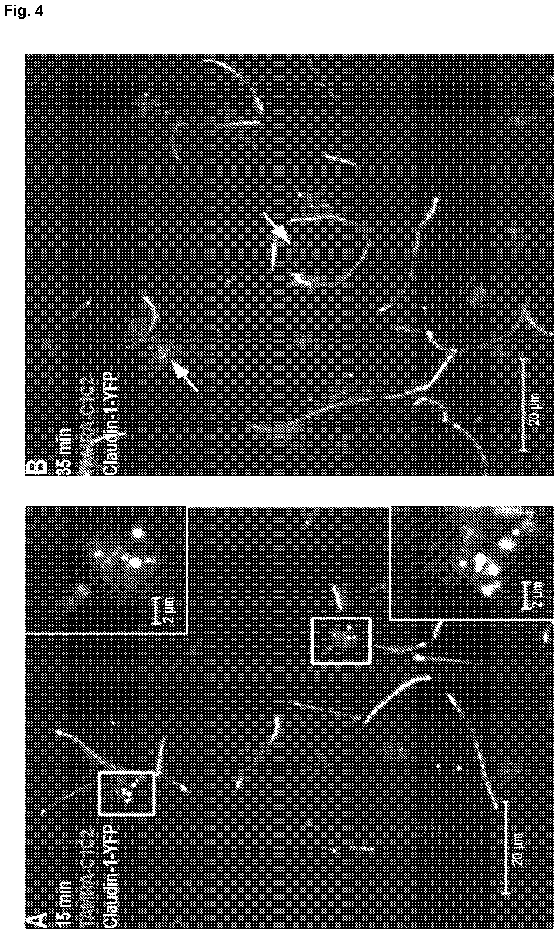

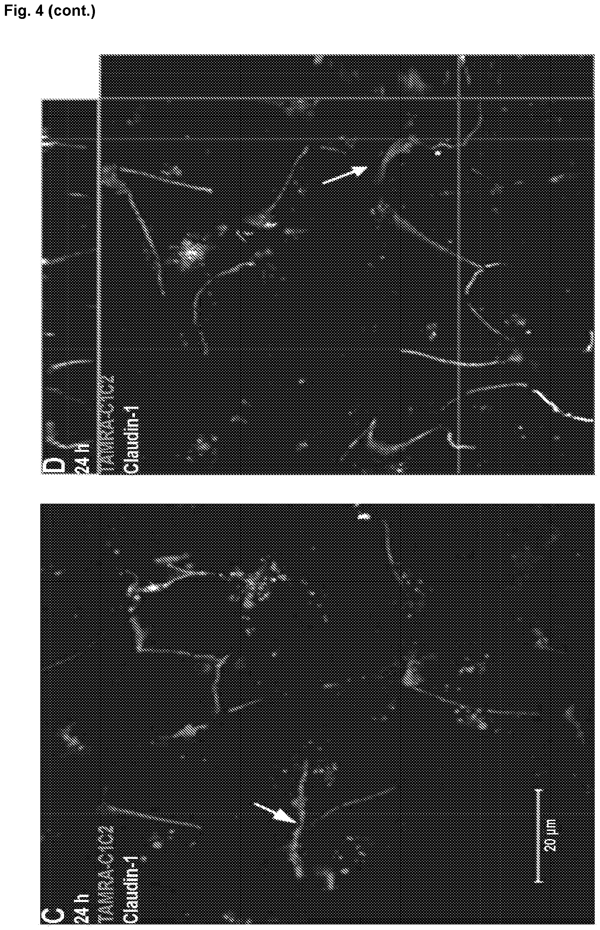

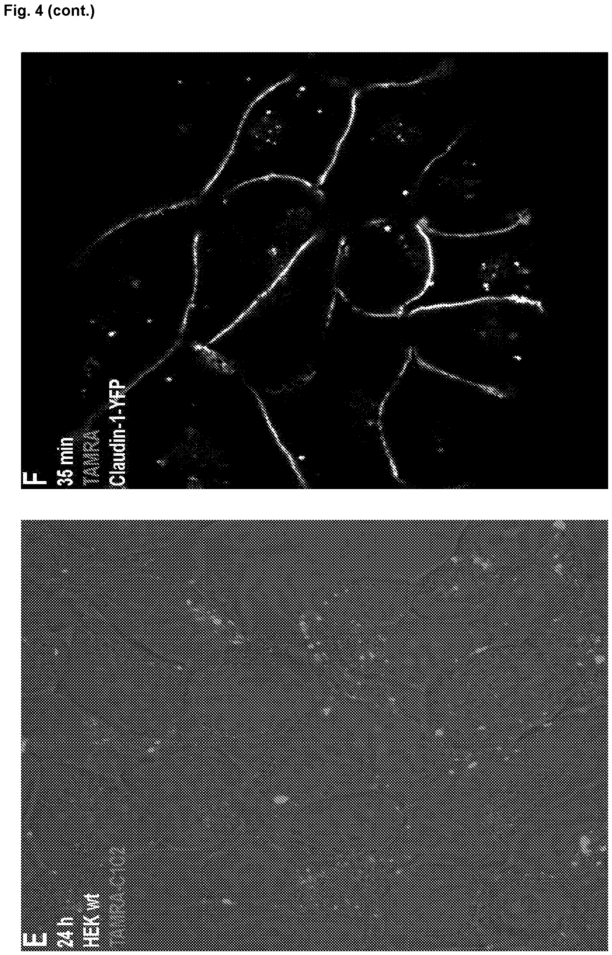

**Fig. 4** **Internalization of TAMRA-C1C2.** (A-F) Human embryonic kidney (HEK)-293 wild type (wt) cells and HEK-293 cells stably transfected claudin-1-yellow fluorescence protein (YFP) were investigated. Cells were treated with TAMRA-C1C2 (40 µM, A-E) or TAMRA (40 µM, F) only for 15 min (A), 35 min (B, F), and 24 h (C-E), respectively. Pictures show a time-dependent increase of TAMRA-C1C2 internalization (red). (A-B) A vesicle-like distribution of TAMRA-C1C2 co-localized with vesicle-like distributed claudin-1-YFP (yellow; arrow). Claudin-1-YFP was mainly membrane-localized at cell-cell contacts. (C-D) TAMRA-C1C2 was localized within the membrane of cell-cell contacts (arrow) and in vesicle-like structures. Expression of claudin-1-YFP in the membrane decreased. (D) Z-stack (xy, quadrat; xz, top; yz, images side). (E) HEK-293 wt cells showing a small amount of unspecific internalized TAMRA-C1C2. (F) TAMRA alone was not able to reach the cytosol.

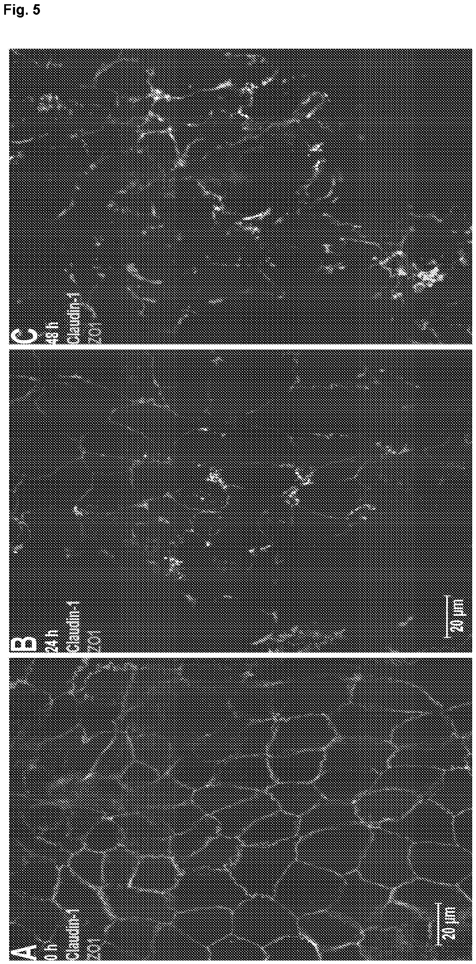

**Fig. 5** **C1C2-induced decrease of membrane-localized claudin-1.** (A-C) Immunofluorescence of claudin-1 (green) and ZO-1 (red) of Caco-2 cell monolayers 0 h (A), 24 h (B), and 48 h (C) after C1C2 (300 µM) treatment, respectively. (A) Mainly cell membrane- and co-localized immunoreactivity of claudin-1 and ZO-1. (B-C) A significant decrease of membrane-localized claudin-1 and inhomogeneous distribution of claudin-1 and ZO-1 was observed after C1 C2 treatment. Representative examples are shown (n=?).

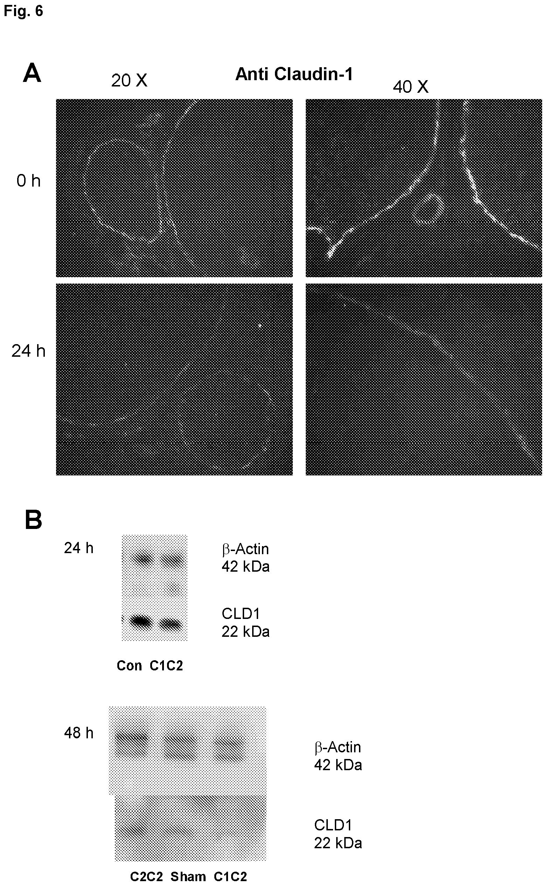

**Fig. 6** **Perineurial C1 C2 treatment decreases claudin-1 in the perineurium of rats.** (A) Rats were injected perineurially with C1C2 (400 µM) as described. Tissue from sciatic nerve was harvested and stained with anti-claudin-1 antibody after the indicated time points. Reduced immunoreactivity was seen 24 h after C1C2 injection. (B) Western Blot analysis of the expression of claudin-1 after C1 C2 treatment of rats: Rats were perineurially injected with C1 C2 (400 µM) or solvent only (sham) or C2C2 (400 µM). Control animal received no treatment (Con). After 24 h or 48 h the sciatic nerve was obtained. Claudin-1 expression decreased in the membrane fraction at both time points. Representative examples are shown (n=3).

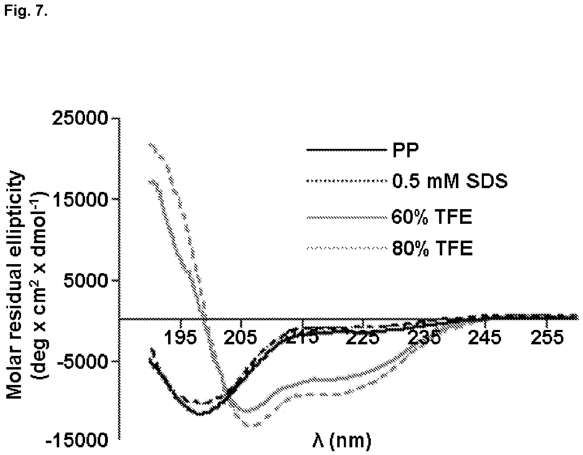

**Fig. 7** **Structural Behaviour of C2C2.** CD (circular dichroism) spectroscopy of C2C2 (50 µM) in different solvents. Random coil of C2C2 in phosphate puffer (PP, 10 mM, pH 7.4) with the characteristic minimum at ~200 nm. Random coil of C2C2 in PP and 0.5 mM SDS (sodium dodecyl sulphate). Helical conformation of C2C2 in PP and 60% and 80% TFE (2,2,2-trifluoroethanol) with the characteristic minima at 222 and 210-208 nm.

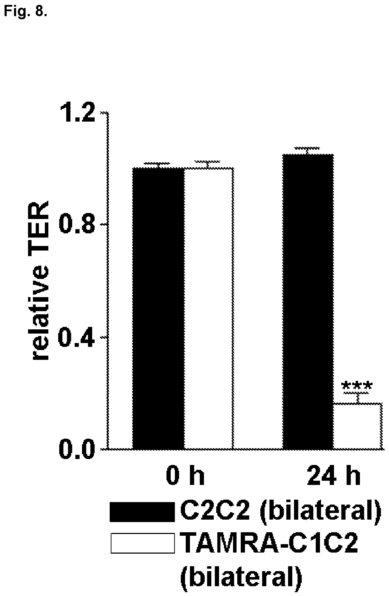

**Fig. 8** **TAMRA-C1C2 but not C2C2 transiently modulate the paracellular permeability of Caco-2 cell monolayers.** Cells were grown on 24-well filters. Reduction of the TER after bilateral application of 300 µM TAMRA-C1C2 and no reduction of the TER after bilateral application of 300 µM C2C2. Data are represented as mean ± SEM; n=6; ***p< 0.0001 (Mann Withney test, one-tailed).

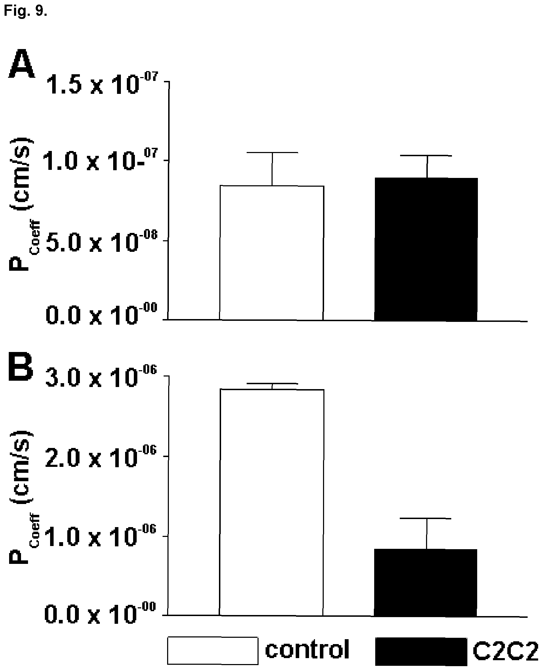

**Fig. 9** **No increase in the permeation of FD-10 nor Lucifer Yellow of Caco-2 cell monolayers by C2C2.** Cells were grown on 24-well filters. Determination of the P_{Coeff} (pemeation coefficient; cm/s) of (A) FD-10 (10 kDa) and (B) Lucifer Yellow (0.4 kDa) of control treated and C2C2 treated cell monolayers. The paracellular permeability was determined 48 h after C2C2 treatment. No significant change of the paracellular permeation of FD-1 0 of C2C2 treated Caco-2 cell monolayers. Data are represented as mean ± SEM; n=6.

### EXAMPLES

The invention is further described by the following examples. These are not intended to limit the scope of the invention. The experimental examples relate, in part, to various in vitro and in vivo experiments carried out in cell lines, including human cell lines, in addition to animal models, including rat models. The cell lines and models represent relevant models used to demonstrate the invention by way of example. The invention is relevant for all mammals, especially humans, who are intended subjects for application of the subject matter of the present invention.

The examples of the invention demonstrate that the peptides of the present invention 1) increase the paracellular flux of ions, high, and low molecular weight compounds and decrease the membrane localization of claudin-1 in Caco-2 cells; 2) internalize and co-localize with claudin-1 in claudin-1-YFP transfected HEK-293 cells and 3) facilitate antinociception by opioids or sodium channel blockers and decrease claudin-1 expression in the perineurium of peripheral nerves in rats.

**Design and Structural Behavior of Claudin-derived Peptides.** The 29 amino acid peptide C1C2 is derived from the C-terminal part of the ECL1 of rat claudin-1, claudin-1⁵³⁻⁸¹. We introduced three specific modifications as compared to the native peptide sequence. The two cysteines Cys⁵⁴ and Cys⁶⁴ were substituted by serines, whereas the C-terminal Arg⁸¹ was amidated. Figure 1*A* shows the design and amino acid sequence of C1C2. To investigate the cellular pathway, the peptide was N-terminally labeled with TAMRA ((5(6)-carboxytetramethylrhodamine). The peptide C2C2 from the C-terminal part of the ECL1 of claudin-2, rat claudin-2⁵³⁻⁸¹, which contains the same modifications as C1C2, was applied as negative control. Peptides were synthesized by SPPS (solid phase peptide synthesis) using the Fmoc (9-fluorenylmethoxycarbonyl)/*t*-Bu strategy. After purification by preparative HPLC (high performance liquid chromatography), peptides were characterized using analytical RP (reversed phase)-HPLC and identified by ESI-MS (electrospray ionisation mass spectrometry). Table 1 summarizes the claudin-derived peptides.

**Table 1. Claudin-derived peptides modulating the paracellular barrier**

| Peptide | Sequence | MW[Da] | Paracellular Permeability |
|---|---|---|---|
| C1C2 | SSVSQSTGQIQSKVFDSLLNLNSTLQATR-**NH₂** | 3107.6 | ↑ |
| TAMRA-C1C2 | TAMRA-S**S**VSQSTGQIQ**S**KVFDSLLNLNSTLQATR-**NH₂** | 3519.6 | ↑ |
| C2C2 | E**S**ATHSTGITQ**S**DIYSTLLGLPADIQAAQ-**NH₂** | 2996.5 | Ø |

| | | | |
|---|---|---|---|
| bold: mutations of amino acid residues; TAMRA: 5(6)-carboxytetramethylrhodamine; CF; ↑ : increase; -: no change | | | |

We studied the secondary structure of the peptides in different environments using CD (circular dichroism) spectroscopy (Fig. 1*B).* At first, the conformations were analyzed in phosphate buffer (PP, 10 mM, pH 7.4) in which C1C2 showed no defined secondary structure (random coil) with the characteristic minimum at ~200 nm. The same result could be obtained for C2C2 (Fig. 7). Otherwise, in micelles of SDS (sodium dodecyl sulphate) a pronounced β-sheet conformation was found for C1C2, which can be described by the maximum at 190-200 nm and the minimum at 225-210 nm, whereas C2C2 showed random coil as in PP (Fig. 7). In 60% TFE (2,2,2-trifluoroethanol) C1C2 was mainly helical, but 80% TFE showed still a higher percentage of helicity. Same results could be obtained for C2C2 (Fig. 7).

**C1C2 Transiently Modulates the Paracellular Permeability of Caco-2 Cell Monolayers. A** significant reduction of the transcellular electrical resistance (TER) was found 24 h after bilateral C1C2 (300 µM) treatment (Fig. 2A). The same result was observed for the TAMRA-labeled C1C2 which confirmed that TAMRA did not influences C1C2 efficacy (Fig. 8). Control cells showed stable TER-values (Fig. 2A). C2C2 treated cells were unaffected (Fig. 8). An increase of the barrier integrity was found after removing C1 C2 from the cell monolayer. Complete recovery of the cell monolayer could be observed 24 h later, i.e. after 72 h (Fig. 2A). Apical treatment showed also a significant reduction of the TER, but less efficient then the bilateral treatment (Fig. 2*B*). Treatment with C1C2 increased the permeation of FD-10 (fluorescein isothiocyanate dextran) and Lucifer Yellow, which resulted in 10 to 20-fold lower permeation coefficients as compared to control treated cells (Fig. 2 *C*,*D*). Peptide C2C2 did not influence the paracellular permeation of these agents (Fig. 9).

**C1C2 increases the mechanical nociceptive thresholds of claudin-1 expressing sciatic nerves *in vivo.*** Immunoreactivity in sciatic nerves from naïve rats for claudin-1 was observed in the perineurium as well as in adjacent vessels (Fig. 3A). No staining was seen in the sciatic nerve itself or in the endoneurium. For *in vivo* functional studies, C1 C2 as well as C2C2 and solvent control were injected perineurially at 0 h at the sciatic nerve. In addition DAMGO or TTX were applied in a perisicatic injection 0-4 d after the injection of the peptide. Mechanical nociceptive thresholds (paw pressure threshold, PPT) after injection of DAMGO or TTX were unchanged in solvent as well as in C2C2-treated control rats (Fig. 3*B*-*D*). In contrast, an increase of mechanical nociceptive thresholds was observed 24 h after injection of 100 µM C1 C2 peptide and 10 min after 30 µg DAMGO. Significant increases of PPT were obtained 48 h after the injection of 400 µM of C1C2 and 10 min after 30 µg DAMGO. No change in mechanical nociceptive thresholds was seen before the injection of DAMGO (Baseline: control solvent: 0 h 71.17±2.13 g; 24 h 68.0±3.85 g; 48 h 64.00±1.92 g; 72 h 69.3±3.08 g; 96 h 66.67±1.25 g. Treated 400 µM C1C2: 0 h 77.67±1.8 g; 24 h 74.67±3.00 g; 48 h 68.0±2.02 g; 72 h 69.00±1.861 g; 96 h 73.3±2.87 g) nor in contralateral paws (Control solvent: 0 h 72.67±1.74 g; 24 h 67.67±3.32 g; 48 h 69.0±1.61 g; 72 h 69.33±3.61 g; 96 h 65.0±3.23 g. Treated 400 µM C1C2: 0 h 77.33±1.3 g; 24 h 63.33±2.36 g; 48 h 67.67±2.03 g; 72 h 65.0±1.25 g; 96 h 67.67±2.09 g) or when DAMGO was injected subcutaneously (7). In a second step TTX, a voltage gated sodium channel blocker was employed, which does not readily penetrate the perineurium under normal conditions using a 10 µM solution. Pretreatment with C1 C2 injected perineurially for 24-48 h enhanced the effect of TTX and allowed for TTX to increase mechanical nociceptive thresholds (Fig. 3*D*).

**C1C2 Internalizes into HEK-293 Claudin-1-YFP Cells and Decreases Membrane-localized Claudin-1 in Caco-2 Cell Monolayers.** For the internalization, HEK-293 (human embryonic kidney) cells stably expressing claudin-1-YFP (yellow fluorescence protein) were treated with TAMRA-C1C2. We visualized TAMRA-C1C2 15, 35 min, and 24 h after peptide administration, respectively (Fig. 4*A*-*D*). The peptide internalized, followed by a clear vesicle-like distribution of TAMRA-C1C2. Claudin-1-YFP was mainly located at cell-cell contacts and partly in vesicle-like structures. Intracellular TAMRA-C1C2 and claudin-1-YFP were mainly co-localized. Then, 24 h after TAMRA-C1C2 treatment a decrease of membrane-localized claudin-1-YFP could be detected and low amounts of TAMRA-C1C2 appeared at the cell membrane. To exclude the possibility that TAMRA alone is internalizing into HEK-293 cells, stably claudin-1-YFP expressing HEK-293 cells were treated with TAMRA, which did not reach the cytosol (Fig. 4*F*). A weak unspecific internalization of TAMRA-C1C2 was observed in wild type HEK-293 cells (Fig. 4*E*).

To investigate the effect of C1 C2 on the distribution of claudin-1, immunofluorescence studies of Caco-2 cell monolayers were performed. Claudin-1 as well as ZO-1 (zonula occludens) were expressed in adjacent membranes. Co-localization of both tight junction proteins was visualized. Data show a clear decrease of membrane-localized claudin-1 and an inhomogeneous cellular distribution of claudin-1 and ZO-1 24 and 48 h after C1C2 treatment (Fig. 5).

**C1C2 Binds to the Membrane and Decreases Membrane-localized Claudin-1 of the Perineurium of Rats.** Claudin-1 immunoreactivity expression was seen in the perineurium of the cross sections in the sciatic nerve in both nerve fascicles after separation of these. After *in vivo* perineurial injection of C1 C2, claudin-1 immunoreactivity decreased after 24 h (Fig. 6A). Similarly, claudin-1 protein expression decreased in the membrane 24 h and 48 h after C1 C2 injection. No change was seen in the cytosol.

### Materials and Methods used in the examples:

**Peptide Synthesis.** Peptides were synthesized with an automated multiple peptide synthesis robot system (Milligen 9050, Milligen, Biosearch, Burlington, MA) by Fmoc/t-Bu strategy using the TentaGel S RAM resin (0.24 mmol/g, 0.5 g). For amino acid coupling the N^{α}-Fmoc-protected amino acid (1 mmol) was activated *in situ* with HBTU (O-benzotriazole-*N*,*N*,*N',N'*-tetramethyl-uronium-hexafluoro-phosphate) (1 mmol) and HOBt (N-hydroxybenzotriacole) (1 mmol). Coupling was achieved within 20 min and repeated once. The Fmoc-group was cleaved in piperidine (20%) in DMF (*N*,*N*-dimethylformamide) for 10 min. The cleavage from the resin and of the side-chain protecting groups was performed in TFA (trifluoroacetic acid) (5% H₂O) for 3 h at room temperature. Peptides were purified by a preparative RP-HPLC system (reversed-phase high performance liquid chromatography) (Shimadzu HPLC System, PolyEncap column, A300, 10 µm, 250 x 20 mm (Bischoff Analysentechnik GmbH, Leonberg and Shimadzu Europe GmbH, Duisburg), flow rate of 10 ml/min). The characterization was performed by analytical RP-HPLC (Shimadzu HPLC System, PolyEncap column, A300, 8 µm, 250 x 4 mm, flow rate of 1 ml/min) and the identity was confirmed by ESI (electrospray ionization) mass spectrometry (TSQ 700, Finnigan MAT 95).

For microscopy studies the claudin-1 derived peptide C1 C2 was N-terminally labeled with TAMRA (5(6)-carboxytetramethylrhodamine) to obtain TAMRA-C1C2 *(protocol).*

The following peptides were synthesized: C1C2 and TAMRA-C1C2, derived from the claudin-1 as well as C2C2, derived from the claudin-2. Peptide sequences and molecular weights are presented in Table 1. Peptides were dissolved in the necessary medium except for TAMRA-C1C2, which was dissolved in a solution containing 0.1% dimethyl sulfoxide (DMSO) or pluronic ®F-68 (0.0012%).

**CD Spectroscopy.** For CD (circular dichroism) spectroscopy peptides (50 µM) were dissolved in phosphate buffer (PP, 10 mM, pH 7.4), PP containing 60% or 80% TFE (2,2,2,-trifluoroethanol) and PP containing 0.5 mM SDS (sodium dodecyl sulphate). Measurements were performed using a J-720 spectrometer (Jasco, Tokyo, Japan) with 0.1 cm silica cuvettes at room temperature. Typical parameters were the scan of the wave length region between 185 and 265 nm and the accumulation of 6 spectra. The baseline of the solvent was subtracted from the peptide spectra. Spectra are presented as molar residual ellipticity of the wave length scan.

**Cell Culture.** Caco-2 (colorectal adenocarcinoma) cells (41) were grown in a humidified atmosphere at 37 °C and 10% CO₂, whereas HEK-293 (human embryonic kidney) cells (42) and HEK-293 cells stably transfected with claudin-1-YFP (yellow fluorescence protein) (43) were grown at 37 °C and 5% CO₂. Cells were cultured in DMEM (Dulbecco's modified Eagle's medium) containing 10% fetal calf serum, 100 units/ml penicillin, 100 µg/ml streptomycin, and 1% L-analyl-L-glutamine.

**Animals.** Animal protocols were approved by the animal care committee of the Regierung von Unterfranken and are in accordance with the International Association for the Study of Pain (44). Male Wistar rats weighing 180-220 g were used.

**Perineurial Injection at the Sciatic Nerve.** Nociceptive inputs from the rat hind paw travels through the sciatic nerve. Under brief isoflurane anesthesia, the right sciatic nerve was located using a 22 G needle connected to a nerve stimulator (Stimuplex Dig RC; Braun, Melsungen, Germany) as previously described (45). The needle was advanced to the point of maximal stimulation at 0.2 mA. 300 µl of the indicated substances was injected. If more than one injection within half hour was necessary the total injected volume was maintained at 300 µl.

Peptides were dissolved in 0.9% NaCl and injected perineurially in the indicated concentrations. Measurements (nociceptive thresholds, tissue harvest for Western blot or immunohistochemistry) were undertaken 24-96 h after peptide injection.

**Measurements of Transcellular Electrical Resistance (TER) and Paracellular Permeability.** 24-well transwell filters (Millicell-CM, Millipore, Eschborn, Germany) were coated with rat tail collagen (250 µg/cm²) for 16 h, washed twice with PBS (phosphate buffer saline) and preincubated with **DMEM** (600 µl basolateral, 400 µl apical for Endohm electrodes and 400 µl basolateral, 500 µl apical for the cellZscope system (nanoAnalytics GmbH, Münster, Germany)) for 24 h. Caco-2 cells (200.000 cell/cm²) were seeded into the filters and grown to high-resistance TER (> 500 Ω/cm²) for approx. 4 days. Medium was changed every day.

The experiments were performed as followed: After reaching high and stable TER values, cell monolayers were treated with peptides as described above. Control cells were incubated with respective medium. The TER values were measured at distinct time intervals. For re-arrangement of the monolayer, C1C2 was removed from the Caco-2 cells by exchange of the medium.

The permeation studies were performed by addition of 25 µg/ml Lucifer Yellow (0.4 kDa) or FD-10 (fluorescein isothiocyanate-dextran, 10 kDa) in HBSS (Hank's balanced salt solution buffer) on the apical side and HBSS alone on the basolateral side. After 10 min, 100 µl probes were removed from the basolateral side and the fluorescence units were measured by a fluorescence plate reader (Tecan, Crailsheim, Germany) to calculate the P_{Coeff} (permeation coefficient, cm/s). The obtained data were analyzed with GraphPad Prism 3.0 program (GraphPad Software, San Diego, USA).

**Confocal Microscopy.** HEK-293 cells and stably claudin-1-YFP expressing HEK-293 cells were transferred into 6-well plates containing collagen-coated cover-slips as described above and grown to 60-70% confluence. Then, cells were treated with the TAMRA-labeled peptide (40 µM) for 15 min, 35 min, and 24 h, respectively. Pluronic ®F-68 (0.0012%) was added as solution enhancer. After removing of the peptide, cells were analyzed with the LSM 510 Meta confocal microscope (Zeiss, Jena, Germany).

**Immunocytochemistry.** Immunocytochemistry was performed as previously described (46). Caco-2 cells were transferred into 6-well plates containing collagen-coated cover-slips and grown to 60-70% confluence before use. Cells were treated with C1C2 (300 µM) as well as the control treated without peptide for 24 and 48 h, respectively. The medium was changed, followed by incubation with rabbit anti-claudin-1 and rabbit anti-ZO-1 (zonula occludens protein) antibodies and incubated with Alexa-Fluor 488-conjugated goat anti-rabbit secondary antibody. Cells were analyzed with the confocal microscope.

The sciatic nerve was embedded in tissue-Tek compound (O.C.T., Miles Inc. Elkhart, Indiana) for claudin-1 staining. Sections (7-10 □m) were mounted onto gelatin-coated slides. Sections were fixed with acetone for 10 min at 4°C, washed in 0.01 M PBS (pH 7.4) for 10 min, incubated for 120 min in PBS containing 1% Triton X-100, washed in 0.01 M PBS and incubated for 30 min in 3% bovine serum albumin plus 1% normal goat serum blocking solution to prevent nonspecific binding. After incubation with rabbit polyclonal primary antibodies against claudin-1 (Invitrogen, Carlsbad, CA, USA) over night at 4°C, sections were incubated with the secondary antibody (donkey anti-rabbit-Alexa Fluor 488 1:600, Invitrogen) dissolved in PBS for 1 h. Finally, the sections were mounted and analyzed using the laser scanning microscope (Zeiss, Jena, Germany).

**Western Blot Analysis.** Sciatic nerves after indicated treatments were taken and homogenized in lysis buffer for Triton X-100 soluble proteins (25 mM TRIS pH 7.6, 120 mM NaCl, 2 mM EDTA, 25 mM NaF, 1% Triton X 100) containing protease inhibitors (Complete, Boehringer, Mannheim, Germany). Cytosol fractions were obtained by homogenization with minipistil and sonification (3 x 5 s/3 s breaks), followed by a centrifugation at 200 *g* for 5 min and subsequent centrifugation of the remaining supernatant at 20,000 *g* for 60 min. The Triton X-100-insoluble pellet containing membrane fraction was resuspended in an equal volume of extraction buffer (25 mM HEPES pH 7.6, 2 mM EDTA, 25 mM NaF, 1% SDS). Extracted protein was diluted in lysis buffer and quantified using the BCA protein assay reagent (Pierce, Rockford, IL, USA) and a plate reader (Tecan, Grödig, Austria). Aliquots of protein were mixed with SDS containing buffer (Laemmli), denatured at 95°C for 5 min, fractionated on SDS polyacrylamide gels and subsequently blotted onto PVDF membranes (PerkinElmer, Boston, MA, USA). Proteins were detected using specific antibodies against claudin-1 (Invitrogen, Carlsbad, CA, USA) and as protein loading control □-actin (Sigma Aldrich, Taufkirchen, Germany), respectively. Visualization was done by luminescence.

**Measurements of Nociceptive Thresholds.** Mechanical thresholds were determined using the paw pressure algesiometer (modified Randall-Selitto test; Ugo Basile, Comerio, Italy) as described before (47). The pressure required to elicit paw withdrawal, the paw pressure threshold (PPT), was determined in blinded experiments. Averages were calculated from three measurements each. An increase in PPT was interpreted as antinociception (analgesia), a decrease in PPT was interpreted as hyperalgesia (pain). Measurements were taken 10-60 min after injection of 100 µl containing 30 µg DAMGO or 10 µM TTX. The optimal doses of DAMGO or TTX have been evaluated in pilot studies (data not shown), but are similar to doses used for intraplantar injection in rats.

**Statistical Analysis.** Results are shown as mean ± S.E.P-values are obtained by the Mann Whitney test, one-tailed. Data were tested for normality and for equal variance. Normally distributed data were analyzed by t-test. Otherwise, the Mann-Whitney rank sum test was used. Multiple measurements were compared by one way ANOVA or by one way ANOVA on ranks in case of not normally distributed data. The post hoc comparisons were performed by Dunnett's or Dunn's method, respectively. Differences were considered significant if p<0.05.

### References

1. Furuse M, Hirase T, Itoh M, Nagafuchi A, Yonemura S, Tsukita S & Tsukita S (1993) Occludin: a novel integral membrane protein localizing at tight junctions. J Cell Biol 123: 1777-1788.
2. Morita K, Furuse M, Fujimoto K & Tsukita S (1999) Claudin multigene family encoding four-transmembrane domain protein components of tight junction strands. Proc Natl Acad Sci U S A 96: 511-516.
3. Ikenouchi J, Furuse M, Furuse K, Sasaki H, Tsukita S & Tsukita S (2005) Tricellulin constitutes a novel barrier at tricellular contacts of epithelial cells. J Cell Biol 171: 939-945.
4. Wu J, Helftenbein G, Koslowski M, Sahin U & Tureci O (2006) Identification of new claudin family members by a novel PSI-BLAST based approach with enhanced specificity. Proteins 65: 808-815.
5. Furuse M, Sasaki H, Fujimoto K & Tsukita S (1998) A single gene product, claudin-1 or-2, reconstitutes tight junction strands and recruits occludin in fibroblasts. J Cell Biol 143: 391-401.
6. Colegio O R, Van Itallie C, Rahner C & Anderson J M (2003) Claudin extracellular domains determine paracellular charge selectivity and resistance but not tight junction fibril architecture. Am J Physiol Cell Physiol 284: C1346-1354.
7. Antonijevic I, Mousa S A, Schafer M & Stein C (1995) Perineurial defect and peripheral opioid analgesia in inflammation. J Neurosci 15: 165-172.
8. Cuschieri J, Gourlay D, Garcia I, Jelacic S & Maier R V (2002) Hypertonic preconditioning inhibits macrophage responsiveness to endotoxin. J Immunol 168: 1389-1396.
9. Junger W G, Hoyt D B, Davis R E, Herdon-Remelius C, Namiki S, Junger H, Loomis W & Altman A (1998) Hypertonicity regulates the function of human neutrophils by modulating chemoattractant receptor signaling and activating mitogen-activated protein kinase p38. J Clin Invest 101: 2768-2779.
10. Simons E J, Bellas E, Lawlor M W & Kohane D S (2009) Effect of chemical permeation enhancers on nerve blockade. Mol Pharm 6: 265-273.
11. Wong V & Gumbiner B M (1997) A synthetic peptide corresponding to the extracellular domain of occludin perturbs the tight junction permeability barrier. J Cell Biol 136: 399-409.
12. Vietor I, Bader T, Paiha K & Huber L A (2001) Perturbation of the tight junction permeability barrier by occludin loop peptides activates beta-catenin/TCF/LEF-mediated transcription. EMBO Rep 2: 306-312.
13. Wong C H, Mruk D D, Lee W M & Cheng C Y (2007) Targeted and reversible disruption of the blood-testis barrier by an FSH mutant-occludin peptide conjugate. Faseb J 21: 438-448.
14. Mrsny R J, Brown G T, Gerner-Smidt K, Buret A G, Meddings J B, Quan C, Koval M & Nusrat A (2008) A key claudin extracellular loop domain is critical for epithelial barrier integrity. Am J Pathol 172: 905-915.
15. Fujita K, Katahira J, Horiguchi Y, Sonoda N, Furuse M & Tsukita S (2000) Clostridium perfringens enterotoxin binds to the second extracellular loop of claudin-3, a tight junction integral membrane protein. FEBS Lett 476: 258-261.
16. Sonoda N, Furuse M, Sasaki H, Yonemura S, Katahira J, Horiguchi Y & Tsukita S (1999) Clostridium perfringens enterotoxin fragment removes specific claudins from tight junction strands: Evidence for direct involvement of claudins in tight junction barrier. J Cell Biol 147: 195-204.
17. Kondoh M, Masuyama A, Takahashi A, Asano N, Mizuguchi H, Koizumi N, Fujii M, Hayakawa T, Horiguchi Y & Watanbe Y (2005) A novel strategy for the enhancement of drug absorption using a claudin modulator. Mol Pharmacol 67: 749-756.
18. Winkler L, Gehring C, Wenzel A, Muller S L, Piehl C, Krause G, Blasig I E & Piontek J (2009) Molecular determinants of the interaction between Clostridium perfringens enterotoxin fragments and claudin-3. J Biol Chem 284: 18863-18872.
19. Pina-Oviedo S & Ortiz-Hidalgo C (2008) The normal and neoplastic perineurium: a review. Adv Anat Pathol 15: 147-164.
20. Kucenas S, Takada N, Park H C, Woodruff E, Broadie K & Appel B (2008) CNS-derived glia ensheath peripheral nerves and mediate motor root development. Nat Neurosci 11: 143-151.
21. Hirakawa H, Okajima S, Nagaoka T, Takamatsu T & Oyamada M (2003) Loss and recovery of the blood-nerve barrier in the rat sciatic nerve after crush injury are associated with expression of intercellular junctional proteins. Exp Cell Res 284: 196-210.
22. Pummi K P, Heape A M, Grenman R A, Peltonen J T & Peltonen S A (2004) Tight junction proteins ZO-1, occludin, and claudins in developing and adult human perineurium. J Histochem Cytochem 52: 1037-1046.
23. Furuse M, Hata M, Furuse K, Yoshida Y, Haratake A, Sugitani Y, Noda T, Kubo A & Tsukita S (2002) Claudin-based tight junctions are crucial for the mammalian epidermal barrier: a lesson from claudin-1-deficient mice. J Cell Biol 156: 1099-1111.
24. Binshtok A M, Bean B P & Woolf C J (2007) Inhibition of nociceptors by TRPV1-mediated entry of impermeant sodium channel blockers. Nature 449: 607-610.
25. Zollner C, Shaqura M A, Bopaiah C P, Mousa S, Stein C & Schafer M (2003) Painful inflammation-induced increase in mu-opioid receptor binding and G-protein coupling in primary afferent neurons. Mol Pharmacol 64: 202-210.
26. Labuz D, Schmidt Y, Schreiter A, Rittner H L, Mousa S A & Machelska H (2009) Immune cell-derived opioids protect against neuropathic pain in mice. J Clin Invest 119: 278-286.
27. Krause G, Winkler L, Mueller S L, Haseloff R F, Piontek J & Blasig I E (2008) Structure and function of claudins. Biochim Biophys Acta 1778: 631-645.
28. Blasig I E, Bellmann C, Cording J, Vecchio G D, Zwanziger D, Huber O & Haseloff R F Occludin Protein Family: Oxidative Stress and Reducing Conditions. Antioxid Redox Signal*.*
29. Takahashi A, Kondoh M, Masuyama A, Fujii M, Mizuguchi H, Horiguchi Y & Watanabe Y (2005) Role of C-terminal regions of the C-terminal fragment of Clostridium perfringens enterotoxin in its interaction with claudin-4. J Control Release 108: 56-62.
30. Wen H, Watry D D, Marcondes M C & Fox H S (2004) Selective decrease in paracellular conductance of tight junctions: role of the first extracellular domain of claudin-5. Mol Cell Biol 24: 8408-8417.
31. Amasheh S, Meiri N, Gitter A H, Schoneberg T, Mankertz J, Schulzke J D & Fromm M (2002) Claudin-2 expression induces cation-selective channels in tight junctions of epithelial cells. J Cell Sci 115: 4969-4976.
32. Fujita T, Kawahara I, Quan Y, Hattori K, Takenaka K, Muranishi S & Yamamoto A (1998) Permeability characteristics of tetragastrins across intestinal membranes using the Caco-2 monolayer system: comparison between acylation and application of protease inhibitors. Pharm Res 15: 1387-1392.
33. Hamamoto K, Kida Y, Zhang Y, Shimizu T & Kuwano K (2002) Antimicrobial activity and stability to proteolysis of small linear cationic peptides with D-amino acid substitutions. Microbiol Immunol 46: 741-749.
34. Fried M W, Shiffman M L, Reddy K R, Smith C, Marinos G, Goncales F L, Jr., Haussinger D, Diago M, Carosi G, Dhumeaux D, Craxi A, Lin A, Hoffman J & Yu J (2002) Peginterferon alfa-2a plus ribavirin for chronic hepatitis C virus infection. N Engl J Med 347: 975-982.
35. Manns M P, McHutchison J G, Gordon S C, Rustgi V K, Shiffman M, Reindollar R, Goodman Z D, Koury K, Ling M & Albrecht J K (2001) Peginterferon alfa-2b plus ribavirin compared with interferon alfa-2b plus ribavirin for initial treatment of chronic hepatitis C: a randomised trial. Lancet 358: 958-965.
36. Ohki S, Marcus E, Sukumaran D K & Arnold K (1994) Interaction of melittin with lipid membranes. Biochim Biophys Acta 1194: 223-232.
37. Weerasuriya A, Rapoport S I & Taylor R E (1979) Modification of permeability of frog perineurium to [14C]-sucrose by stretch and hypertonicity. Brain Res 173: 503-512.
38. Todd B A, Inman C, Sedgwick E M & Abbott N J (2000) Ionic permeability of the frog sciatic nerve perineurium: parallel studies of potassium and lanthanum penetration using electrophysiological and electron microscopic techniques. J Neurocytol 29: 551-567.
39. Grant G J, Vermeulen K, Zakowski M I & Langerman L (2001) Perineural antinociceptive effect of opioids in a rat model. Acta Anaesthesiol Scand 45: 906-910.
40. Kanda T, Numata Y & Mizusawa H (2004) Chronic inflammatory demyelinating polyneuropathy: decreased claudin-5 and relocated ZO-1. J Neurol Neurosurg Psychiatry 75: 765-769.
41. Rousset M, Chevalier G, Rousset J P, Dussaulx E & Zweibaum A (1979) Presence and cell growth-related variations of glycogen in human colorectal adenocarcinoma cell lines in culture. Cancer Res 39: 531-534.
42. Graham F L, Smiley J, Russell W C & Nairn R (1977) Characteristics of a human cell line transformed by DNA from human adenovirus type 5. J Gen Virol 36: 59-74.
43. Cukierman L, Meertens L, Bertaux C, Kajumo F & Dragic T (2009) Residues in a highly conserved claudin-1 motif are required for hepatitis C virus entry and mediate the formation of cell-cell contacts. J Virol 83: 5477-5484.
44. Zimmermann M (1983) Ethical guidelines for investigations of experimental pain in conscious animals. Pain 16: 109-110.
45. Puehler W, Zollner C, Brack A, Shaqura M A, Krause H, Schafer M & Stein C (2004) Rapid upregulation of mu opioid receptor mRNA in dorsal root ganglia in response to peripheral inflammation depends on neuronal conduction. Neuroscience 129: 473-479.
46. Blasig I E, Winkler L, Lassowski B, Mueller S L, Zuleger N, Krause E, Krause G, Gast K, Kolbe M & Piontek J (2006) On the self-association potential of transmembrane tight junction proteins. Cell Mol Life Sci 63: 505-514.
47. Stein C, Gramsch C & Herz A (1990) Intrinsic mechanisms of antinociception in inflammation: local opioid receptors and beta-endorphin. J Neurosci 10: 1292-1298.
48. Sagie and Kohane et al. 2010 PNAS 107:3740-3745).

## Claims

1. Isolated peptide comprising an amino acid sequence of or derived from a claudin protein for use as a pharmaceutical agent in pain therapy.

2. Isolated peptide according to claim 1, **characterised in that** the peptide exhibits an amino acid sequence of or derived from the first extra-cellular loop of a claudin protein.

3. Isolated peptide, preferably according to any one of the previous claims, **characterised in that** the peptide exhibits an amino acid sequence comprising
- a sequence according to
SEQ ID NO. 1 (X₁X₂VX₃QSTGX₄X₅QX₆KVX₇DSLLX₈LX₉X₁₀X₁₁R), whereby X₁ to X₁₁ can be any amino acid,
- a sequence according to SEQ ID NO. 1, whereby X₁=S, N; X₂=S, C; X₃=S, V; X₄=Q, H; X₅=l, M; X₆=S, C; X₇=F, Y; X₈=N, A; X₉=S, N, P; X₁₀=S, Q, T and X₁₁=T, D.
- a sequence according to
SEQ ID NO. 2 (SSVSQSTGQIQSKVFDSLLNLNSTLQATR)
SEQ ID NO. 3 (SCVSQSTGQIQCKVFDSLLNLSSTR),
SEQ ID NO. 4 (SCVVQSTGQMQCKVYDSLLALPQDR),
SEQ ID NO. 5 (SCVVQSTGQMQCKVYDSLLALPQDR),
SEQ ID NO. 6 (NCVVQSTGQMQCKVYDSLLALPQDR),
SEQ ID NO. 7 (NCVVQSTGQMQCKVYDSLLALPQDR),
SEQ ID NO. 8 (SCASQSTGQVQCKLYDSLLALDGHR),
SEQ ID NO. 9 (SCVVQSTGHMQCKVYDSVLALSTER),
SEQ ID NO. 10 (DCVTQSTGMMSCKMYDSVLALSAAR),
SEQ ID NO. 11 (NCVRQANIRMQCKIYDSLLALSPDR) and/or
SEQ ID NO. 12 (ECVWHSTGIYQCQIYRSLLALPQDR),
- a sequence that exhibits any number of conservative amino acid substitutions in comparison to a sequence according to any one of SEQ ID NO. 1 to 12, and/or
- a sequence that exhibits more than 80%, preferably more than 90%, sequence similarity to a sequence according to any one of SEQ ID NO. 1 to 12.

4. Isolated peptide according to the preceding claim, **characterized in that** the peptide comprises a beta-sheet secondary structure.

5. Isolated peptide according to any one of the preceding claims for use in opening the perineurium, dissociating the tight junctions of the perineurium and/or increasing paracellular permeability, preferably of ions, small molecules, pharmaceutical agents and/or high or low molecular weight compounds, through the perineurium.

6. Isolated peptide according to any one of the preceding claims for use as an adjuvant for enhanced delivery and/or transport of pharmaceutical agents across one or more neurological barriers, preferably perineural delivery of pharmaceutical agents to peripheral nerve cells.

7. Isolated peptide according to the preceding claim, **characterised in that** the pharmaceutical agent is a painkiller, such as one or more analgesics and/or anesthetics, preferably opioids, such as morphine or DAMGO, or sodium channel blockers, such as alkaloid based toxins, for example tetrodotoxin (TTX) and/or saxitoxin (STX).

8. Isolated peptide according to any one of the preceding claims for use as an adjuvant in the treatment of pain, preferably nociceptive pain.

9. Isolated peptide according to any one of the preceding claims for use as an adjuvant for painkiller administration, such as analgesics and/or anesthetics, preferably opioids, such as morphine or DAMGO, or sodium channel blockers, such as alkaloid based toxins, for example tetrodotoxin (TTX) and/or saxitoxin (STX), thereby enhancing painkiller function.

10. Isolated peptide according to any one of the preceding claims for use as an adjuvant in the treatment of perioperative acute or chronic pain, such as pain associated with joint replacement or other operations involving hips, shoulders, hands, feet and/or knees, complex regional pain syndrome (CRPS) and/or pain associated with ischemic disease, such as peripheral artery occlusive disease.

11. Isolated peptide according to any one of the preceding claims for use as an adjuvant for enhanced perineural delivery of therapeutic gene vectors across the perineurium, for example enhanced delivery of viral gene vectors, which preferably encode for growth factors or stimulators of cellular regeneration in the treatment of nerve damage.

12. Claudin protein as a target for pain therapy.

13. Pharmaceutical composition comprising the peptide according to any one claims 1 to 11 with a pharmaceutically acceptable carrier material.

14. Pharmaceutical composition according to the preceding claim comprising additionally one or more painkillers, such as analgesics and/or anesthetics, preferably opioids, such as morphine or DAMGO, or sodium channel blockers, such as alkaloid based toxins, for example tetrodotoxin (TTX) and/or saxitoxin (STX).

15. Kit comprising the peptide according to any one of the claims 1 to 11 and/or the pharmaceutical composition according to any one of claims 13 or 14 in addition to a painkiller, which are physically separated and can be mixed shortly prior to administration.

16. Isolated peptide according to any one of claims 1 to 11 and/or the pharmaceutical composition according to any one of claims 13 or 14 for local and/or regional application, preferably for local treatment of pain in a subject by injection or topical application.

17. Isolated peptide according to any one of claims 1 to 11 for use as an adjuvant in the treatment of pain, **characterised in that** patient motor skills are maintained during pain treatment.

18. Isolated peptide according to any one of claims 1 to 11 for use in treatment of any medical condition in which opening the perineurium, dissociating the tight junctions of the perineurium and/or increasing paracellular permeability, preferably of ions, small molecules, pharmaceutical agents and/or high or low molecular weight compounds, through the perineurium during treatment, would be of benefit to a patient.

19. Nucleic acid molecule encoding the amino acid sequence for the peptide according to any one of claims 1 to 11.

20. Gene expression vector comprising the nucleic acid molecule according to the preceding claim.

21. Cell comprising the vector and/or nucleic acid according to any one of claims 19 or 20.

22. Method for enhanced perineural delivery of pharmaceutical agents to peripheral nerve cells comprising administration of the peptide according to any one of claims 1 to 11 and/or the pharmaceutical composition according to any one of claims 13 or 14 with a pharmaceutical agent.

23. Method for the treatment of pain comprising administration of an effective amount of the peptide according to any one of claims 1 to 11 and/or the pharmaceutical composition according to any one of claims 13 or 14 as an adjuvant for painkiller administration, such as administration of analgesics and/or anesthetics, preferably opioids, such as morphine or DAMGO, or sodium channel blockers, such as alkaloid based toxins, for example tetrodotoxin (TTX) and/or saxitoxin (STX), thereby enhancing painkiller function, to a subject in need of pain treatment.


SEQUENCE LISTING

<110>  Leibniz-Institut für Molekulare Pharmakologie
       Universität Würzburg
       Blasig, Ingolf
       Zwanziger, Denise
       Rittner, Heike

<120>  PEPTIDE ADJUVANT FOR IMPROVED PERIPHERAL ANALGESIA

<130>  XII 652/11

<160>  13

<170>  PatentIn version 3.5

<210>  1
<211>  25
<212>  PRT
<213>  Artificial

<220>
<223>  synthetic sequence


<220>
<221>  misc_feature
<222>  (1)..(2)
<223>  Xaa can be any naturally occurring amino acid

<220>
<221>  misc_feature
<222>  (4)..(4)
<223>  Xaa can be any naturally occurring amino acid

<220>
<221>  misc_feature
<222>  (9)..(10)
<223>  Xaa can be any naturally occurring amino acid

<220>
<221>  misc_feature
<222>  (12)..(12)
<223>  Xaa can be any naturally occurring amino acid

<220>
<221>  misc_feature
<222>  (15)..(15)
<223>  Xaa can be any naturally occurring amino acid

<220>
<221>  misc_feature
<222>  (20)..(20)
<223>  Xaa can be any naturally occurring amino acid

<220>
<221>  misc_feature
<222>  (22)..(24)
<223>  Xaa can be any naturally occurring amino acid

<400>  1

Xaa Xaa Val Xaa Gln Ser Thr Gly Xaa Xaa Gln Xaa Lys Val Xaa Asp
1               5                   10                  15


Ser Leu Leu Xaa Leu Xaa Xaa Xaa Arg
            20                  25


<210>  2
<211>  29
<212>  PRT
<213>  Artificial

<220>
<223>  synthtic sequence

<400>  2

Ser Ser Val Ser Gln Ser Thr Gly Gln Ile Gln Ser Lys Val Phe Asp
1               5                   10                  15

Ser Leu Leu Asn Leu Asn Ser Thr Leu Gln Ala Thr Arg
            20                  25

<210>  3
<211>  25
<212>  PRT
<213>  Homo sapiens

<400>  3

Ser Cys Val Ser Gln Ser Thr Gly Gln Ile Gln Cys Lys Val Phe Asp
1               5                   10                  15

Ser Leu Leu Asn Leu Ser Ser Thr Arg
            20                  25

<210>  4
<211>  25
<212>  PRT
<213>  Homo sapiens

<400>  4

Ser Cys Val Val Gln Ser Thr Gly Gln Met Gln Cys Lys Val Tyr Asp
1               5                   10                  15

Ser Leu Leu Ala Leu Pro Gln Asp Arg
            20                  25

<210>  5
<211>  25
<212>  PRT
<213>  Homo sapiens

<400>  5

Ser Cys Val Val Gln Ser Thr Gly Gln Met Gln Cys Lys Val Tyr Asp
1               5                   10                  15

Ser Leu Leu Ala Leu Pro Gln Asp Arg
            20                  25

<210>  6
<211>  25
<212>  PRT
<213>  Homo sapiens

<400>  6

Asn Cys Val Val Gln Ser Thr Gly Gln Met Gln Cys Lys Val Tyr Asp
1               5                   10                  15

Ser Leu Leu Ala Leu Pro Gln Asp Arg
            20                  25

<210>  7
<211>  25

<212>  PRT
<213>  Homo sapiens

<400>  7

Asn Cys Val Val Gln Ser Thr Gly Gln Met Gln Cys Lys Val Tyr Asp
1               5                   10                  15

Ser Leu Leu Ala Leu Pro Gln Asp Arg
            20                  25

<210>  8
<211>  25
<212>  PRT
<213>  Homo sapiens

<400>  8

Ser Cys Ala Ser Gln Ser Thr Gly Gln Val Gln Cys Lys Leu Tyr Asp
1               5                   10                  15

Ser Leu Leu Ala Leu Asp Gly His Arg
            20                  25

<210>  9
<211>  25
<212>  PRT
<213>  Homo sapiens

<400>  9

Ser Cys Val Val Gln Ser Thr Gly His Met Gln Cys Lys Val Tyr Asp
1               5                   10                  15

Ser Val Leu Ala Leu Ser Thr Glu Arg
            20                  25

<210>  10
<211>  25
<212>  PRT
<213>  Homo sapiens

<400>  10

Asp Cys Val Thr Gln Ser Thr Gly Met Met Ser Cys Lys Met Tyr Asp
1               5                   10                  15

Ser Val Leu Ala Leu Ser Ala Ala Arg
            20                  25

<210>  11
<211>  25
<212>  PRT
<213>  Homo sapiens

<400>  11

Asn Cys Val Arg Gln Ala Asn Ile Arg Met Gln Cys Lys Ile Tyr Asp
1               5                   10                  15

Ser Leu Leu Ala Leu Ser Pro Asp Arg
            20                  25

<210>  12
<211>  25

<212>  PRT
<213>  Homo sapiens

<400>  12

Glu Cys Val Trp His Ser Thr Gly Ile Tyr Gln Cys Gln Ile Tyr Arg
1               5                   10                  15

Ser Leu Leu Ala Leu Pro Gln Asp Arg
            20                  25

<210>  13
<211>  29
<212>  PRT
<213>  Artificial

<220>
<223>  synthetic sequence

<400>  13

Glu Ser Ala Thr His Ser Thr Gly Ile Thr Gln Ser Asp Ile Tyr Ser
1               5                   10                  15

Thr Leu Leu Gly Leu Pro Ala Asp Ile Gln Ala Ala Gln
            20                  25